# EUROPEAN PATENT SPECIFICATION

(11) **EP 2 820 422 B1**
(45) Date of publication and mention of the grant of the patent: **22.02.2017**
(21) Application number: 13712598.5
(22) Date of filing: 28.02.2013
(51) Int. Cl.: G01N 33/566

(54) **ABCG2 TRANSPORTER ASSAY**
ABCG2-TRANSPORTERTEST
ANAYSE DE TRANSPORTEUR ABCG2

(30) Priority: 28.02.2012 HU P1200131; 28.02.2012 US 201261603981 P
(43) Date of publication of application: 07.01.2015
(73) Proprietor: Cellpharma LLC, 1113 Budapest (HU)
(72) Inventor: SARKADI, Balázs, 1121 Budapest (HU); TELBISZ, Ágnes, 1082 Budapest (HU)
(74) Representative: Svingor, Adam
(86) International application number: PCT/HU2013/000021
(87) International publication number: WO 2013/128217

(56) References cited:
- WO-A2-2007/132279
- VAIDYA SONIYA S ET AL: "Lack of interaction between tauroursodeoxycholate and ATP-binding cassette transporter isoform G2 ( ABCG2)", MOLECULAR PHARMACEUTICS, vol. 3, no. 3, June 2006 (2006-06), pages 303-306, XP008162398, ISSN: 1543-8384
- POZZA A ET AL: "Overexpression of homogeneous and active ABCG2 in insect cells", PROTEIN EXPRESSION AND PURIFICATION, ACADEMIC PRESS, SAN DIEGO, CA, vol. 63, no. 2, 1 February 2009 (2009-02-01), pages 75-83, XP025686801, ISSN: 1046-5928, DOI: 10.1016/J.PEP.2008.09.021 [retrieved on 2008-10-21]
- DEZI M ET AL: "The multidrug resistance half-transporter ABCG2 is purified as a tetramer upon selective extraction from membranes", BIOCHIMICA ET BIOPHYSICA ACTA (BBA) - BIOMEMBRANES, ELSEVIER, AMSTERDAM, NL, vol. 1798, no. 11, 1 November 2010 (2010-11-01), pages 2094-2101, XP027274136, ISSN: 0005-2736 [retrieved on 2010-08-04]

### SEQUENCE LISTING

<110> Sarkadi, Balazs
   Telbisz, Agnes
<120> ABC TRANSPORTER ASSAY
<130> 110348
<160> 2
<170> PatentIn version 3.5
<210> 1
   <211> 90
   <212> DNA
   <213> Artificial Sequence
<220>
   <223> Nucleotide sequence encoding the N-terminal sequence of the His6 tagged ABCG2
<400> 1
   catcatcatc atcatcacgg aaggagaagg gccagtgttg cggcgggaat tttggtccct 60
   cgtggaagcc caggactcga tggcatatat 90
<210> 2
   <211> 30
   <212> PRT
   <213> Artificial Sequence
<220>
   <223> The N-terminal sequence of the His6 tagged ABCG2
<220>
   <221> MISC_FEATURE
   <222> (1)..(6)
   <223> Polyhistidine tag
<220>
   <221> MISC_FEATURE
   <222> (9)..(14)
   <223> Phosphorylation site
<220>
   <221> MISC_FEATURE
   <222> (18)..(23)
   <223> Thrombin cleavage site
<400> 2
   His His His His His His Gly Arg Arg Arg Ala Ser Val Ala Ala Gly
   1               5                   10                  15
   Ile Leu Val Pro Arg Gly Ser Pro Gly Leu Asp Gly Ile Tyr
               20                  25                  30

## Description

The invention relates to a membrane preparation for use in an increased sensitivity ABCG2 protein activity assay, said preparation comprising ABCG2 protein in lipid membranes and one or more bile acid compounds. Said preparation can be a cell membrane preparation or a reconstituted membrane protein preparation. The invention also relates to methods for obtaining said preparations, either cell membrane preparations or, preferably, membrane protein preparations comprising a reconstituted, recombinant ABCG2 protein. The invention also relates to kits comprising said preparations as well as uses said kits, membrane preparations or bile acid compounds in high sensitivity ABCG2 protein activity assays.

### BACKGROUND OF THE INVENTION

Several ABC transporters have important role in general drug metabolism. These multidrug resistance ABC (MDR-ABC) transporters actively extrude various hydrophobic or amphipathic, but otherwise chemically unrelated compounds from the cells. Due to their localization at important tissue barriers ABC-MDR transporters protect the body against toxic materials, and profoundly alter pharmacokinetics of several drugs. Therefore it has a great importance to examine the interaction of xenobiotics, drugs, and drug candidates with the MDR-ABC transporters.

For example, a group of ABC transporters (P-gp/ABCB1, several MRP/ABCC, BCRP/ABCG2) plays a significant role in the multidrug resistance observed in cancer cells. These multidrug (MDR) ABC transporters can lower drug concentrations inside the cancer cells below effective levels due to outward drug transport based on ATP cleavage. MDR-ABC transporters also have an important role at cellular and tissue barriers by protecting the body and its cells against xenobiotics and drugs. Due to their role in cancer multidrug resistance and modifying the ADME-tox features of several drugs it is of particular importance to characterize the interactions of these MDR-ABC transporters with xenobiotics, drugs, and drug candidates.

MDR-ABC transporters overexpressed in eukaryote cells provide a possibility to test such interactions, by measuring drug-stimulated ATPase activity or direct drug transport in isolated membrane preparations. The ATPase activity of the MDR-ABC transporters is measured as a vanadate sensitive ATPase activity in the presence of inhibitors of other ATPases in the insect cell membrane preparation.

In this test system MDR-ABC transporters show a characteristic ligand-independent basal ATPase activity, which can be either stimulated or inhibited by transporter-interacting drugs. The molecular background of this basal activity is not known. It may be either an uncoupled (transport independent) ATPase activity, or may be caused by the presence of unknown substrate(s), in the membrane compartment. The relatively high basal ATPase activity, especially in the case of ABCG2, decreases the sensitivity of the ATPase assays because the effects of the stimulatory drugs, relative to this basal activity, is relatively low.

Attempts have been made in the art to prepare pure ABCG2 membrane preparations useful among others in ABCG2 functional or activity assays. In several cases the preparation, though being pure, proved to be of impaired activity. Published trials to purify recombinant ABCG2 protein from insect cell in a few cases used metal chelate cromatography but due to the harsh conditions they applied the resulted protein preparation had only a very faint activity not suitable to carry out functional assays for testing drug interactions. Moreover, it has also not been suggested that in the particular method after the chromatography step the protein is to be reconstituted directly in the environment of the assay by using a bacterial or mammalian lipid extract.

Jacobs, A et al. (2011) have devised a methodology to recombinant synthesis of human ABCG2 expressed in the yeast Saccharomyces. They were able to show substrate stimulation, but in this work authors used yeast strain for expression of a mutant variant of ABCG2. For the solubilisation of the membrane protein a detergent was used but no lipid was utilized. McDevitt, C. A. et al (2006) have obtained an inactivated preparation due to the use of an inactivating detergent. Pozza A et al. (2009) who expressed ABCG2 in insect cells have not applied either a lipid or a detergent upon solubilization and have obtained a very low activity preparation (0.357 umol ATP/min/mg) and Dezi et al. reached an even lower activity (0.006 umol ATP/min/mg).

Velamakanni S. et al. ["A functional steroid-binding element in an ATP-binding cassette multidrug transporter" Molecular Pharmacology 73 12-17 (2008)] identified a steroid binding element in the membrane domain of ABCG2 and suggested that further studies are required to compare steroid interactions and their effect on oligomerization of ABCG2. The authors speculate upon whether cholesterol acts as a competitive substrate or might enhance the basal ATPase activity of ABCG2 R482G variant.

Imai Y et al. ["Breast Cancer Resistance Protein Exports Sulfated Estrogens but Not Free Extrogens" Molecular Pharmacology 64, 610-618 (2003)] teach that sulphated steroids (estrogens) inhibit ABCG2 mediated drug transport and probably drug-stimulated ATPase activity of ABCG2.

Pavek P et al. ["Human Cancer Resistance Protein: Interactions with Steroid Drugs, Hormones, the Dietary Carcinogen 2-Amino-1-methyl-6-phenylimidazo(4,5-b)pyridine, and Transport of Cimetidine" 312, 144-152 (2005)] teach that 17-beta-estradiol is a potent inhibitor of ABCG2.

Vaidya S et al. (2006) in their work disclose elements of a kit for assessing ABCG2 together with bile acids taurodeoxycholate (TUDC), this kit is used in a different context, namely to ability of ABCG2 to transport TUDC across a membrane. However, the authors have found no interaction between ABCG2 and TUDC. Moreover, the authors state that TUDC does not affect ABCG2-mediated transport of estradiol-glucoronid.

It was also described, somewhat contrary to the results of Velamakanni et al, that the drug-stimulated ABCG2-ATPase and the related drug transport activity are greatly increased in the presence of increasing membrane cholesterol content (WO2007/132279). The Sf9 insect cell membrane has a relatively low cholesterol content, thus cholesterol loading increased the sensitivity of the ATPase and transport assay for characterizing drug interactions. Cholesterol loading of the Sf9 cell membranes thus mimicks the mammalian cell membrane, and the activity of the ABCG2 protein requires this cholesterol composition.

Though an increase in substrate transport activity has been achieved by loading the membranes with cholesterol, the problem of high basal ATPase activity has not been solved in the art so far.

None the above publications suggest any method or solution to improve sensitivity of ABCG2 substrate transport assays.

While the prior art suggested that certain steroid compounds may increase basal ATPase activity of ABCG2 (c.f. Velamakanni S. et al. 2008) The present inventors have unexpectedly found that a group of compounds including bile acids and related compounds are useful in lowering the basal ATPase activity in membranes comprising ABCG2, e.g. in isolated ABCG2 protein preparations and in isolated membranes overexpressing ABCG2.

Moreover, the present Inventors have developed a new methodology to prepare and reconstitute isolated MDR-ABC transporter proteins with full activity and refined the ATPase assay to provide a high-sensitivity screening system. Thereby membrane protein preparations comprising active and reconstituted ABCG2 can be prepared and utilized in order to further increase the value of such assays.

### BRIEF DESCRIPTION OF THE INVENTION

The invention relates to the following embodiments:
Use of a kit in an increased sensitivity ABCG2 protein activity assay, said kit comprising
   - a membrane preparation or liposomes, said membranes or liposomes comprising a recombinant ABCG2 transporter protein capable of transporting an ABCG2 substrate compound across the membrane and showing substrate-stimulated ATPase activity,
   - one or more bile acid compounds,
wherein said membrane preparation is a cell membrane preparation or a membrane protein preparation reconstituted in liposomes, wherein the basal ATPase activity of the ABCG2 protein is reduced in said assay in comparison with a control not comprising said one or more bile acid compounds.

Preferably, said membrane preparation is a cell membrane preparation obtainable from cells expressing said recombinant ABCG2,
said preparation comprising a dimeric ABCG2 having a substrate transport activity.

Preferably, said membrane preparation is a membrane protein preparation comprising a reconstituted ABCG2 protein. More preferably, said membrane protein preparation is obtainable by a method comprising the following steps:
- providing or preparing an expression vector suitable for expressing a nucleic acid in a host, said nucleic acid comprising a nucleotide sequence encoding an ABCG2 protein and, operably linked thereto a nucleotide sequence encoding a peptide tag for protein purification.
- overexpressing said nucleic acid sequence in a host to obtain a tagged protein comprising the ABCG2 protein and the peptide tag, wherein said ABCG2 protein is functional,
- obtaining the expressed tagged protein in a solubilized form,
- the solubilized tagged protein is subjected to purification comprising affinity chromatography step specific for the peptide tag to obtain a purified protein,
- the purified protein is reconstituted into an appropriate lipid environment for regaining function, preferably into liposomes,
wherein
the affinity chromatograpy is metal chelate chromatograpy and
the peptide tag is capable of complexing the metal ion of the metal chelate chromatography and is engineered to the N-terminus of the ABCG2 protein for allowing purification.

The invention also relates to a use of a membrane preparation in an increased sensitivity ABCG2 protein activity assay, said preparation comprising
- liposomes, said liposomes comprising a recombinant ABCG2 transporter protein capable of transporting an ABCG2 substrate compound across the membrane of said liposomes and showing substrate-stimulated ATPase activity,
- one or more bile acid compounds,
wherein the level of said bile acid derivative in said preparation is below the critical micelle forming concentration (CMC) and
wherein said membrane preparation is a cell membrane preparation or a membrane protein preparation, and wherein the basal ATPase activity of the ABCG2 protein is reduced in said assay in comparison with a control not comprising said one or more bile acid compounds.

Preferably, the basal ATPase activity of the membrane preparation is lower than the basal ATPase activity of a control preparation lacking said one or more added bile acid compounds or comprising a lower level thereof.

In a further aspect, the invention relates to an ABCG2 protein activity assay method comprising the steps of
- providing a membrane preparation as defined in any of claims 6) to 7), said membrane preparation comprising one or more bile acid compounds
- assessing the activity of the ABCG2 protein present in the membrane preparation, wherein
   the basal ATPase activity of the ABCG2 protein is reduced in comparison with a control not comprising said one or more bile acid compounds.

In a further aspect the invention also relates to a use of a bile acid compound for reducing basal ATPase activity of a membrane preparation comprising a recombinant ABCG2 transporter protein, capable of transporting an ABCG2 substrate compound across the membrane,
wherein said membrane preparation is a cell membrane preparation or a membrane protein preparation reconstituted in liposomes.

In a further aspect the invention also relates to a use of a membrane preparation as defined herein in an ABCG2 protein activity assay wherein the basal ATPase activity of the ABCG2 protein is reduced in said assay in comparison with a control not comprising said one or more bile acid compounds wherein
the one or more bile acid compound are selected from cholic acid, chenodeoxycholic acid, glycocholic acid, taurocholic acid, deoxycholic acid, lithocholic acid or a derivative thereof which is an amide or an ester of the carboxyl group on the side chain connected to the 17 position of the sterane core or a salt thereof.

In a preferred embodiment any of the uses of the invention comprises the use of an agent for increasing transport activity, preferably cholesterol.

In a preferred embodiment said membrane preparation as defined herein further comprises an agent for increasing transport activity, preferably cholesterol.

In a preferred embodiment said kit according to the invention further comprises an agent for increasing transport activity, preferably cholesterol.

In a further aspect the invention also relates to a method for obtaining an isolated membrane protein preparation for use in an ABCG2 assay according to the invention, said method comprising
- providing or preparing an expression vector suitable for expressing a nucleic acid in a host, said nucleic acid comprising a nucleotide sequence encoding an ABCG2 protein and, operably linked thereto a nucleotide sequence encoding a peptide tag for protein purification.
- overexpressing said nucleic acid sequence in a host to obtain a tagged protein comprising the ABCG2 protein and the peptide tag, wherein said ABCG2 protein is functional,
- obtaining the expressed tagged protein in a solubilized form by
   - preparing cell membrane fraction from the cells of the host, said membranes comprising the tagged protein,
   - solubilizing the tagged protein from the cell membrane fraction by a detergent-lipid mixture whereas the functionality of the ABCG2 transporter is maintained, in a solubilisation buffer by DDM detergent and E.coli lipid mixture and
- the solution fraction is separated to obtain solubilised ABCG2 protein in the supernatant,
- the solubilized tagged protein is subjected to purification comprising affinity chromatography step specific for the peptide tag to obtain a purified protein,
- the purified protein is reconstituted into an appropriate lipid environment for regaining function with preformed liposomes made of a lipid extract, said lipid extract being selected from the group of
   a bacterial lipid extract and/or
   a eukaryotic lipid extract selected from a vertebrate lipid extract, a mammalian lipid extract and a brain lipid extract,
   wherein preferably the reconstitution lipid extract is supplemented with cholesterol, and
- wherein the dimeric form of ABCG2 is maintained and this fact is detected by a method assessing the size of the native proteins, and
wherein
said host is an insect cell,
the affinity chromatograpy is metal chelate chromatograpy and
the peptide tag is capable of complexing the metal ion of the metal chelate chromatography and is engineered to the N-terminus of the ABCG2 protein for allowing purification.

Preferably, the peptide tag is a His-tag.

Preferably, the preformed liposomes in the reconstitution agent are destabilized and the reconstitution agent is incubated with purified ABCG2 protein, wherein preferably the reconstitution lipid extract is supplemented with cholesterol.

Preferably, the expressed tagged protein in a solubilized form comprises
- preparing cell membrane fraction from the cells of the host by
   - disruption of the cells by mechanical homogenization in a hypo-osmotic buffer,
   - removal of cellular debris by low speed centrifugation at 200 to 400 g
   - centrifugation of the supernatant to obtain a membrane pellet at 30 000 to 70 000 g for 30 min to 90 min,
   - homogenization of the membrane pellet,
said membranes comprising the tagged protein,
- solubilizing the tagged protein from the cell membrane fraction by a detergent-lipid mixture whereas the functionality of the ABCG2 transporter is maintained,
- assessing the amount of protein and/or transporter activity in the membrane preparation, and optionally
- washing the pelleted membranes,
- repeatedly pelleting, washing and resuspending the membranes.

Preferably, said membrane preparation is a cell membrane preparation obtainable from cells expressing said recombinant ABCG2 by a method according to any of above methods,
said preparation comprising a dimeric ABCG2 having a substrate transport activity.

### DEFINITIONS

A "bile acid compound" is an amphipatic steroid compound having the sterane core carrying a 3α hydroxyl group, preferably the sterane core of any bile acid, and a side chain at the 17 position (all IUPAC numbering) carrying or terminated in a carboxyl group or an amide or ester thereof. A bile acid compound is capable to be incorporated into micelles or liposomes. A bile acid compound can be natural or artificial. Preferably a bile acid compound is a bile acid, a bile acid derivative or a salt thereof.

Preferably a bile acid compound is a compound according to formula I below. Nevertheless, it should be understood that substituents identical with Q1 and/or Q2 may be present at other positions of the sterane core as well, provided that the function of the bile acid compound is maintained, e.g. in particular at position 16, preferably at the 16 alpha position.

A "bile acid" is understood herein as an end-product of cholesterol catabolism in animals, the main functions of which are to act as detergents or emulsifying agents in the intestines to aid the digestion and absorption of fatty acids, monoacylglycerols and other fatty products and to prevent the precipitation of cholesterol in bile, and having a 3α hydroxyl group and maintaining a side chain connected to the 17 position (17 position side chain), preferably at least a C3, preferably a C5 side chain of the cholesterol. Preferably, the bile acid is bile acid of vertebrate origin, preferably of fish, amphibian, reptile, bird, mammalian, primate or human origin, preferably a bile acid synthesized in the liver (primary bile acid) or a naturally modified variant thereof (secondary bile acid), e.g. by bacteria.

Bile acids are for example cholic acid, chenodeoxycholic acid, glycocholic acid, taurocholic acid, deoxycholic acid, lithocholic acid.

A "bile acid derivative" is a bile acid or a chemically modified bile acid which may be modified at the 17 position side chain by adding a moiety thereto, preferably to the C3 or C5 side chain of the cholesterol. Preferably, a bile acid derivative is an amide or an ester of the carboxyl group on the side chain connected to the 17 position of the sterane core. A bile acid derivative may be modified, e.g. substituted at the sterane core provided that it function as a bile acid is maintained. The bile acid derivative also can be the conjugate of a bile acid or a bile acid compound. In a preferred embodiment the bile acid derivative is a bile acid derivative detergent, preferably , e.g. CHAPS (3-[(3-Cholamidopropyl)dimethylammonio]-1-propanesulfonate), CHAPSO (3-[(3-Cholamidopropyl)dimethylammonio]-2-hydroxy-1-propanesulfonate) or big CHAP (CAS Number: 86303-22-2) or a variant thereof, e.g a deoxy big CHAP.

A "conjugate" is understood herein as a chemical compound that has been formed by joining two or more compounds and thus comprises two or more moieties. Preferably, features and/or functions of each moieties can be distinguished within the conjugate. Typically, in the present invention, one of the moieties is a bile acid derivative and the other moiety is a reporting group, e.g. a fluorescent group.

"ABCG2 protein", which may be a monomer or a dimer, or an "ABCG2 transporter" which is a dimer, is ATP-binding cassette sub-family G member 2 that is encoded by the ABCG2 gene. Preferably the ABCG2 gene is a gene of a vertebrate, preferably a mammal, highly preferably human gene or a variant thereof encoding ABCG2 protein the amino acid sequence of which is identical to any wild type ABCG2 protein in at least 70%, preferably at least 80% or 85%, more preferably at least 90%, 95%, 96%, 97%, 98% or 99%. Preferably the "ABCG2 protein" or the "ABCG2 transporter" is functional. The ABCG2 protein can be a mutant or a variant, preferably which shows the effect of the invention. If a mutant is specified herein it does not exclude that other mutations may be present.

A "functional" ABCG2 transporter is an ABCG2 protein dimer capable of drug stimulated ATPase activity and ATPase coupled drug transport. ATPase coupled drug transport is a drug transport across a membrane, preferably a lipid membrane, e.g. the membrane compartment or a cell membrane. In case of a cell membrane or a membrane compartment wherein the ABCG2 protein is in a normal arrangement the drug is transported from inside to outside, i.e. the drug is extruded from the membrane compartment or the cell. In case of an "inside-out" membrane compartment wherein the ABCG2 protein is in an inverse arrangement the drug is transported from outside to inside of the membrane compartment.

"Functionality" refers to the presence or level of function of an ABCG2 transporter. The functionality of said ABCG2 transporter can be assessed by physical, chemical, biochemical and/or biological method.

An ABC transporter protein has "substrate transport activity" when, under appropriate conditions, it is capable of transporting a substrate of said transporter through the biological membrane in which said transporter is present. Thus, substrate transport activity is an inherent biological property of the ABC transporter protein which is independent from the fact whether it is possible to measure or detect said activity. Substrate transport activity of an ABC transporter protein typically may be detected or assessed by one or more of the following assays: cellular or vesicular transport assay, wherein it is usually possible to directly show the transport of the substrate, or substrate stimulated ATPase assay which is typically, if assay conditions are appropriately set, indicative of substrate transport. Most often vanadate sensitive or inhibitor sensitive ATPase assays are used. Substrate sensitivity specifically relates to activity as measured by a substrate stimulated ATPase assay.

A "membrane compartment" is microscopic formation wherein a lipid membrane, preferably a lipid bilayer, encloses an inner space, a lumen, and thereby separates it from the environment.

A "liposome" is a membrane compartment, preferably a microscopic and artificial membrane compartment having an aqueous lumen enclosed by one or more lipid bilayer, preferably phospholipid bilayer.

A "preparation" is a composition of matter artificially prepared or deliberately prepared by Man preferably according to a pre-defined and/or reproducible method or a protocol. Preferably the preparation has multiple components.

A "membrane preparation" is a preparation comprising lipid membranes, preferably a colloidal preparation wherein membrane compartments are dispersed in an aqueous medium. Said membrane compartments may comprise one or more substance integrated or inserted into the membrane thereof, e.g. one or more membrane protein.

A "cell membrane preparation" is a preparation from tissue or cells, in which the membranous elements, preferably those formed from the cell membrane, are enriched in relation to other cellular constituents. Typically, cell membrane preparations are obtained by disruption of the cells and an isolation method, e.g. differential centrifugation or other technique to isolate a fraction enriched in membranes. Typically, the lipid membrane compartment contains integral membrane proteins of the cell. Cell membrane preparations are the standard biological material for receptor binding assays or transporter assays.

A "membrane protein preparation" is a membrane preparation comprising at least one specific protein or protein of interest integrated into the membrane compartments, preferably into the arteficial liposomes. In a preferred embodiment the protein is a recombinant protein. In a preferred embodiment the protein is purified and reconstituted.

A "control preparation" is a preparation useful for assessing the effect of a set of feature of a given preparation (or preparation of interest). The control preparation differs from the given preparation in a definite set of features wherein the effect of said set of features is to be studied or compared. The set of features can be one feature or multiple features. A feature is definite if it can be detected, measured and/or if it is experimentally added to or achieved or elicited in the preparation. The control preparation can be a control membrane preparation. For example, a control membrane preparation useful for assessing the effect of one or more bile acid compound in a membrane preparation having the same composition except that the control membrane preparation lacks bile acid compounds or comprises a detectably lower level of bile acid compounds.

In a preferred embodiment any one or each of the above preparations used herein or any one or each of the components thereof is "isolated", i.e. its original environment has been artificially changed or changed by Man, preferably replaced by another artificial environment or medium. Preferably said preparation or component is separated from its original environment. Preferably said preparation or component is purified. Purified is to be understood herein as comprising a reduced concentration or activity of membrane transporter proteins or activities other that ABCG2 protein or activity detected by an analytical or functional (assay) method. As used herein percentage of purity is the ratio of the concentration and of the all proteins, membrane proteins or membrane transport proteins as detected by the given analytical method, multiplied by 100; or the ratio of activities of the ABCG2 protein and of all membrane transport proteins or ATPases or ABC proteins or any other relevant activities as measured by the given functional (assay) method, multiplied by 100. Thus, 100% purity is a purity when by the said analytical or functional (assay) method no other membrane transporter protein or no other membrane transporter protein activity can be detected.

The "original level" of a bile acid compound in a preparation is the level before adding said bile acid compound to said preparation. Typically, the original level of a bile acid compound is the level of said bile acid compound measured in a cell membrane preparation or in a membrane protein preparation prepared without the addition of any bile acid compound.

The "physiological" content or level of a compound or a compound family or class, e.g. a bile acid compound in a membrane is the level of said bile acid compound measurable in a membrane of biological origin without any addition of the compound or depletion thereof. While the term physiological level includes levels in membranes *in vivo,* typically physiological levels are measured in biological samples, e.g. in membrane preparation obtained without any artificial or deliberate modification of the level of said compound, e.g. without any addition of a bile acid compound or depletion thereof. Preferably, the physiological content or level is that develops in membrane preparations derived from such cells, with no artificially induced cholesterol loading or depletion, for example during cell culture.

The term "comprises" or "comprising" or "including" are to be construed here as having a non-exhaustive meaning and allow the addition or involvement of further features or method steps or components to anything which comprises the listed features or method steps or components. The expression "consisting essentially of" or "comprising substantially" is to be understood as consisting of mandatory features or method steps or components listed in a list e.g. in a claim whereas allowing to contain additionally other features or method steps or components which do not materially affect the essential characteristics of the use, method, composition or other subject matter. It is to be understood that "comprises" or "comprising" or "including" can be replaced herein by "consisting essentially of" or "comprising substantially" if so required without addition of new matter.

### BRIEF DESCRIPTION OF THE FIGURES

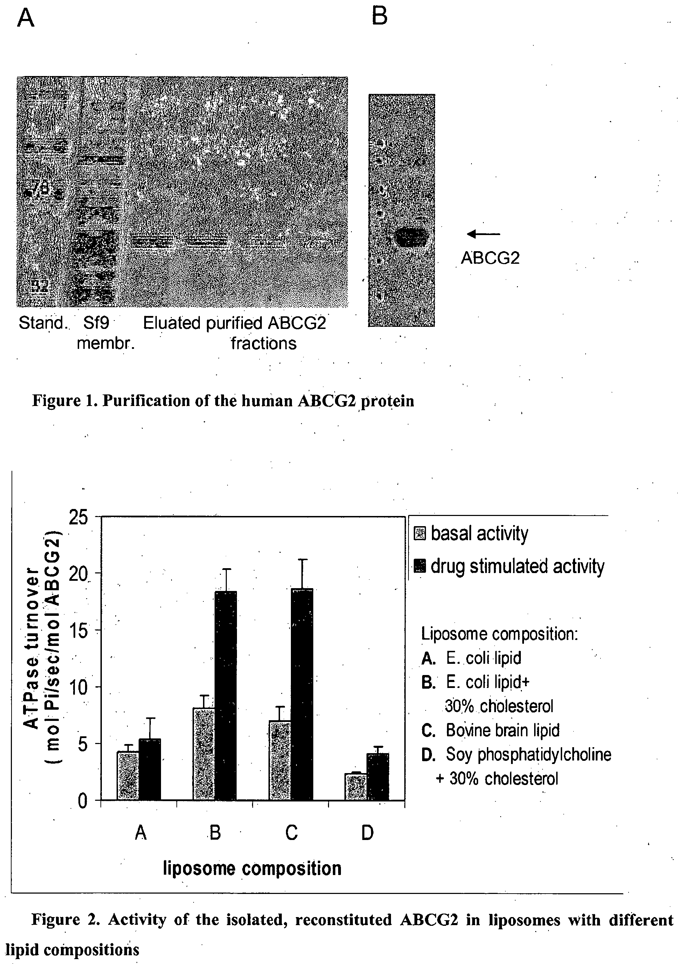

**Figure 1****. Purification of the human ABCG2 protein**
   His6-ABCG2 protein was solubilized from Sf9 membrane by a detergent-lipid mixture and purified by His-tag affinity chromatography (Ni-NTA). Panel A shows original membrane fraction and the eluated fractions of Ni-NTA cromatography, containing the pure ABCG2 protein (Coomassie staining). Panel B shows the identification of the purified ABCG2 protein by BXP-21 antibody on Western blot.
**Figure 2****. Activity of the isolated, reconstituted ABCG2 in liposomes with different lipid compositions**
   ATPase activity is provided in mol Pi/sec/mol ABCG2. Basal (light grey bars) and drug-stimulated (dark grey bars) activity was measured in the presence of 5 uM quercetin, as transported substrate. Lipid composition of liposomes A: E. coli lipid extract, B: E.coli lipid extract + 30% cholesterol, C: bovine brain lipid extract, D: soybean phosphatidycholine + 30% cholesterol (D). For further details see the Methods.

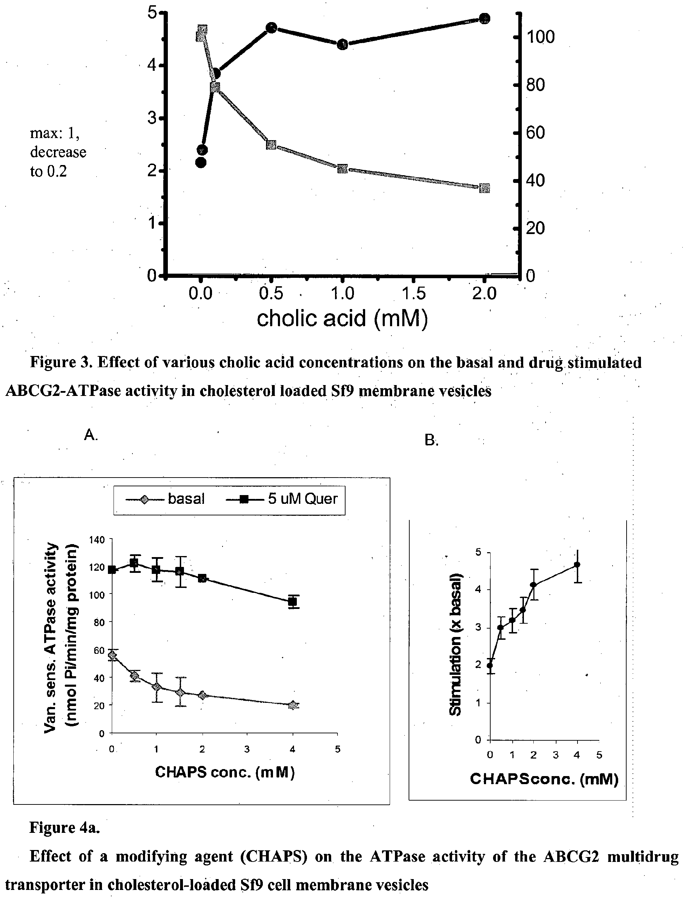

**Figure 3****. Effect of various cholic acid concentrations on the basal and drug stimulated ABCG2-ATPase activity in cholesterol loaded Sf9 membrane vesicles**
   The left ordinate (grey squares) indicates the relative change in the basal ABCG2-ATPase activity. The right ordinate (black circles) shows the relative change in the stimulation of ABCG2-ATPase activity (stimulation was achieved by 5 uM quercetin). Cholic acid concentration is given in millimoles (mM).
**Figure 4a****. Effect of modifying agent (CHAPS) on the ATPase activity of the ABCG2 multidrug transporter in cholesterol-loaded Sf9 cell membrane vesicles**
   Panel A: Modulation of basal (grey diamonds) and substrate-stimulated (black squares) ABCG2-ATPase activity, depending on the concentration of CHAPS. ABCG2-ATPase activity was stimulated by 5 uM quercetin as a substrate. Panel B: Modulation of the relative drug stimulation of the ABCG2-ATPase activity, depending on CHAPS concentration. Drug (5 uM quercetin) stimulatory effect is calculated as the ratio of drug-stimulated and basal activity.
**Figure 4b****. Effect of various CHAPS concentrations on the basal and drug stimulated ABCG2-ATPase activity in cholesterol loaded Sf9 membrane vesicles**
   The left ordinate (grey squares) indicates the relative change in the basal ABCG2-ATPase activity. The right ordinate (black circles) shows the relative change in the stimulation of ABCG2-ATPase activity (stimulation was achieved by 5 uM quercetin). CHAPS concentration is given in millimoles.
**Figure 5****. Effect of an ABCG2 substrate (quercetin) on the ABCG2-ATPase** - **modulation of activity by a modulatory agents (0.1% CHAPS) in cholesterol loaded Sf9 membranes.** Panel A: Stimulation of the ABCG2-ATPase activity by quercetin in the absence (grey squares) and in the presence (light grey circles) of 0.1 % CHAPS, Panel B: Relative stimulation of the ABCG2-ATPase activity by quercetin in the absence (black squares) and in the presence (dark grey circles) of 0,1% CHAPS.
**Figure 6****. Effect of nilotinib on the ABCG2-ATPase activity - modulation by 0.1% CHAPS in cholesterol loaded Sf9 membranes.** Panel A: Effect of nilotinib on the ABCG2-ATPase in the absence and in the presence of 0.1% CHAPS, Panel B: Relative changes in the ABCG2-ATPase activity by nilotinib.
**Figure 7****. Effect of bile acids and derivatives on the ATPase activity of the ABCG2-proteoliposome preparation.** Modulatory effects of several bile acids on the basal and substrate stimulated (5 uM quercetin and 50 uM prazosin) ABCG2-ATPase activity was measured at a selected modifying agent concentrations. These were 0.1% CHAPS, 2 mM glycocholate (GC), 0.5 mM taurodeoxycholate (TDC), 0.25 mM taurocholate (TC), 0.1 mM deoxycholate (DOC), and 2 mM cholic acid (CA). The proteoliposome used for purified ABCG2 reconstitution was composed of bovine brain lipid extract + 40% cholesterol.
**Figure 8****.** The N-terminal sequence (SEQ ID NO: 1) of the His6 tagged ABCG2 and its coding nucleotide sequence (SEQ ID NO: 2). The polyhistidine tag, a phosphorylation site and the thrombin cleavage site are underlined.

### DETAILED DESCRIPTION OF THE INVENTION

The present inventors have developed a high-sensitivity functional assay that can be used both in isolated ABCG2 protein preparations and in isolated membranes overexpressing ABCG2. The key novelty of the modified ATPase assay is that the ligand-sensitivity of the ABCG2-ATPase activity can be greatly enhanced by lowering the drug-independent "baseline" ATPase activity. This is achieved by the addition of "modifying" compounds which are bile acid derivatives. Under these conditions the maximum level of the drug-stimulated ABCG2-ATPase activity is unchanged, whereas the relative drug-stimulated ATPase, thus the sensitivity of the assay is significantly increased.

So as to facilitate and validate the study of ABCG2 as an active transporter, the present inventors have also developed a purification and reconstitution method, which maintains the functionality of the ABCG2 protein. The purified protein, reconstituted in an appropriate lipid environment shows high-level drug-stimulated ATPase activity and active drug transport properties. The purified and reconstituted ABCG2 protein allows to perform both functional and structural studies, exploring ABCG2-drug interactions at the molecular level.

### Kits

The invention relates to the use of a kit in an increased sensitivity ABCG2 protein activity assay as defined in the appended claims.

Preferably, said bile acid compound in the kit is a bile acid or a bile acid derivative or a salt thereof.

In a preferred embodiment the bile acid derivative is a bile acid derivative detergent, preferably a zwitterionic or a non-ionic detergent, e.g. a derivative of the cholic acid. In a preferred embodiment, the bile acid compound is CHAPS, CHAPSO big CHAP or a variant of any of them.

### Membrane preparations

Also disclosed are membrane preparations for use in an increased sensitivity ABCG2 protein activity assay, said preparations comprising
- cell membrane vesicles or liposomes comprising a recombinant ABCG2 transporter protein capable of transporting an ABCG2 substrate compound across the membrane and showing substrate-stimulated ATPase activity,
- a bile acid compound,
wherein the level of said bile acid derivative in said preparation is below the critical micelle forming concentration (CMC) and
wherein said membrane preparation is a cell membrane preparation or a membrane protein preparation. Said bile acid compound is preferably a bile acid or a bile acid derivative and/or a conjugate and/or a salt thereof. Even more preferably, the bile acid compound is a bile acid compound having the general formula I as defined above.

Preferably, in the membrane preparation of the invention the basal ATPase activity of the membrane preparation is lower than the basal ATPase activity of a control preparations lacking said one or more added bile acid compounds or comprising a lower level thereof.

In an embodiment of the invention the membrane preparation is a cell membrane preparation obtainable from cells expressing said recombinant ABCG2.

Preferably, the cell membrane preparation is obtainable by a method disclosed herein, e.g. a method comprising the following steps:
- providing an expression system comprising cells suitable for expressing a recombinant ABCG2 transporter protein,
- expressing said recombinant ABCG2 transporter protein in said cells,
- preparing a membrane preparation comprising said recombinant ABCG2 protein integrated in the membranes of said membrane preparation and
- adding a bile acid compound as defined herein.

In an embodiment of the invention the membrane protein preparation is a membrane protein preparation comprising a reconstituted ABCG2 protein.

Preferably, said membrane protein preparation reconstituted in liposome is obtainable by a method as disclosed herein, e.g. a method comprising the following steps:
- providing an expression system comprising cells suitable for expressing a recombinant ABCG2 transporter protein,
- expressing said recombinant ABCG2 transporter protein in said cells,
- obtaining the expressed ABCG2 transporter protein in a solubilized form,
- the solubilized ABCG2 transporter protein is subjected to purification,
- reconstituting the purified protein into an appropriate lipid environment for regaining function, preferably into liposomes,
- adding a bile acid compound as defined herein.

### Methods for obtaining membrane protein preparations

The isolated membrane protein preparation for use in an ABCG2 assay can be obtained by a method comprising
- providing or preparing an expression vector suitable for expressing a nucleic acid in a host, said nucleic acid comprising a nucleotide sequence encoding an ABCG2 protein and, operably linked thereto (preferably to the 5' end of the ABCG2 coding sequence), a nucleotide sequence encoding a peptide tag for protein purification, preferably a tag allowing or facilitating purification.
- overexpressing said nucleic acid sequence in a host to obtain a tagged protein comprising the ABCG2 protein and the peptide tag, wherein said ABCG2 protein is functional,
- obtaining the expressed tagged protein in a solubilized form,
- the solubilized tagged protein is subjected to purification comprising affinity chromatography step specific for the peptide tag to obtain a purified protein,
- the purified protein is reconstituted into an appropriate lipid environment for regaining function, preferably into liposomes.

This method can be a preferred embodiment of the method for obtaining a membrane preparation as disclosed above.

The tagged protein can be considered as a fusion protein.

In an embodiment of this method obtaining the expressed tagged protein in a solubilized form comprises
- preparing cell membrane fraction from the cells of the host, said membranes comprising the tagged protein,
- solubilizing the tagged protein from the cell membrane fraction by a detergent-lipid mixture whereas the functionality of the ABCG2 transporter is maintained. The skilled person will realize that a detergent which inactivates to protein (like Fc-16) is to be avoided.

Preferably, the preparation of membranes comprises the steps of
- disruption of the cells by mechanical homogenization, preferably in a hypo-osmotic buffer,
- removal of cellular debris by centrifugation, preferably by low speed centrifugation, (g=2-400)
- centrifugation of the supernatant to obtain a membrane pellet (30 000 to 70 000 g, preferably 40 000 to 60 000 g, more preferably 45 000 to 50 000 g for 30 min to 90 min, preferably from 50 min to 70 min, more preferably from 55 min to 66 min),
- homogenization of the membrane pellet,
- optionally washing the pelleted membranes, preferably in a high salt wash buffer (preferably said wash buffer comprising 300 to 700 mM salt, preferably 400 to 600 mM salt, wherein the salt is preferably a metal halogenide, preferably an alkali earth metal halogenide or an alkali halogenide),
- optionally repeatedly pelleting, washing and resuspending the membranes,
- optionally assessing the amount of protein and/or transporter activity in the membrane preparation.

In an embodiment the purification of the ABCG2 protein prior to affinity chromatography comprises the steps of
- solubilisation of the membrane protein in a solubilisation buffer by DDM detergent (1%) and E.coli lipid mixture (0.4%), and
- separation of a solution fraction, preferably centrifugation, to obtain solubilised ABCG2 protein in the supernatant.

In a further embodiment of this method the affinity chromatography purification of the ABCG2 protein comprises the steps of
- application of the solubilised ABCG2 protein to the affinity chromatography medium,
- washing the medium,
- elution of the ABCG2 protein.

Preferably the chromatography medium is a His-binding metal chelate medium. Preferably, elution is carried out by an elevated level of imidazol or by setting the pH level.

In a further embodiment of this method the purification of the ABCG2 protein after the affinity chromatography comprises the step of reconstitution with a reconstitution agent obtaining liposomes, said liposomes comprising the ABCG2 protein.

Preferably, the reconstitution agent is the preformed liposome used for reconstitution made of a lipid extract, preferably a bacterial lipid extract, more preferably an *E. coli* lipid extract and/or a eukaryotic lipid extract, preferably a vertebrate lipid extract, more preferably a mammalian lipid extract, even more preferably a brain lipid extract.

In a preferred embodiment, the reconstitution lipid extract is supplemented with cholesterol. Reconstitution of a fully active ABCG2 is optionally achieved when detergent is removed.

In a variant of this reconstitution method liposomes are formed and purified ABCG2 is added to the liposomes.

In a further variant of this method liposomes in the reconstitution agent are destabilized and the reconstitution agent is incubated with purified ABCG2 protein. The liposomes can be destabilized e.g. by detergent, e.g. by 0.01 % to 5%, preferably 0.1% to 1%, preferably 0.2% to 0.5% detergent, e.g. by Triton-X detergent. Preferably, the reconstitution agent is formed from dried lipids in aqueous solution e.g. by sonication. Preferably, the excess detergent is removed. More preferably, reconstitution of a fully active ABCG2 is achieved when detergent is removed. Detergent can be removed e.g. by hydrophobic adsorption, gel chromatography, dialysis, ion-exchange chromatography or by other adsorbent material. Adsorbent materials, like BioBead® adsorbent (BioRad) or CALBIOSORB™ adsorbent (EMD4Biosciences), are available in the art. In a preferred method the excess detergent is removed e.g. by an adsorbent, e.g. by adsorbent beads.

In the method of the invention the dimeric form of ABCG2 should be maintained. This fact can be detected by any method suitable to assess the size of the native proteins, e.g. native gel electrophoresis, size exclusion chromatography, ultracentrifugation or by detecting activity of the protein.

In a further embodiment of the method for obtaining an isolated membrane protein preparation the affinity chromatograpy is metal chelate chromatograpy and the peptide tag is capable of complexing the metal ion of the metal chelate chromatography. Preferably, the metal chelate chromatograpy is a Ni-chelate chromatography, a Co-chelate chromatography, a Zn-chelate chromatography or a Cu-chelate chromatography.

Preferably, the peptide tag is a His-tag, preferably a cleavable 6 histidine tag (His6-tag).

In a preferred embodiment any peptide tag is engineered to the N-terminus of the ABCG2 protein for allowing purification.

In a preferred embodiment of the purification method the eluted fractions are concentrated in detergent and optionally the concentrated fractions are stored. In a preferred embodiment the purified and reconstituted ABCG2 liposomes are stored. Storage is preferably done below -20°C, more preferably below -60°C or below -70°C or about *at* -80°C.

The present inventor have surprisingly found that the turnover numbers of the ABCG2 preparations obtained by the method according to the invention are approximately an order of magnitude higher than those obtained previously in purified and reconstituted ABCG2 preparations.

### Methods for obtaining cell membrane preparations

A membrane preparation which is a cell membrane preparation for use in an ABCG2 assay preferably with a reduced basal ATPase activity can be obtained by a method comprising
- providing or preparing an expression vector suitable for expressing a nucleic acid in a host, said nucleic acid comprising a nucleotide sequence encoding an ABCG2 protein and, operably linked thereto (preferably to the 5' end of the ABCG2 coding sequence), a nucleotide sequence encoding a peptide tag for protein purification, preferably for allowing, facilitating protein purification,
- overexpressing said nucleic acid sequence in a host to obtain a tagged protein comprising the ABCG2 protein and the peptide tag, wherein said ABCG2 protein is functional,
- preparing a cell membrane preparation from the cells of the host.

In a preferred embodiment of this method the preparation of membranes comprises the steps of
- disruption of the cells by mechanical homogenization, preferably in a hypo-osmotic buffer,
- removal of cellular debris by centrifugation (low speed centrifugation, g=2-400)
- centrifugation of the supernatant to obtain a membrane pellet (30 000 to 70 000 g, preferably 40 000 to 60 000 g, more preferably 45 000 to 50 000 g for 30 min to 90 min, preferably from 50 min to 70 min, more preferably from 55 min to 66 min),
- homogenization of the membrane pellet,
- optionally washing the pelleted membranes, preferably in a high salt wash buffer (preferably comprising 300 to 700 mM salt, preferably 400 to 600 mM salt, wherein the salt is preferably a metal halogenide, preferably an alkali halogenide),
- optionally repeatedly pelleting, washing and resuspending the membranes
- optionally quantitation of the amount of protein and/or transporter activity (def) in the membrane preparation.

In a preferred embodiment of protein expression, the protein is overexpressed in an insect cell, preferably in a baculovirus-Sf9 insect cell system.

### Methods for reducing basal ATPase activity

Also disclosed is a method for reducing basal ATPase activity of a membrane preparation preferably a membrane protein preparation for use in an ABCG2 protein activity assay, said method comprising
- providing a preparation comprising membrane vesicles or liposomes, said membrane vesicles or liposomes comprising recombinant ABCG2 transporter protein capable of transporting an ABCG2 substrate compound across the membrane (of said liposomes) and showing substrate-stimulated ATPase activity,
- adding a bile acid compound as defined herein to the preparation,
or a conjugate of said bile acid compound,
or a salt thereof.

Thus, the bile acid compound level is increased as compared to the original or physiological level of said bile acid compound in the same type of membrane.

Preferably, the ABCG2 protein is purified and reconstituted according to a method disclosed herein.

### ABCG2 protein activity assays

The invention relates to an ABCG2 protein activity assay method comprising the steps of
- providing a membrane preparation as defined herein
- assessing the activity of the ABCG2 protein present in the membrane preparation.

The ABCG2 protein activity assay method, wherein the step of providing a membrane preparation as defined herein comprises the step of providing a membrane preparation as defined above and adding said bile acid compound thereto.

In a preferred embodiment the bile acid compound is added to the membrane preparation during the assay.

In a further embodiment the bile acid compound is added to the membrane preparation prior to the assay.

### Uses

The invention also relates to a use of a bile acid compound as defined herein,
or a conjugate of said bile acid derivative,
or a salt thereof,
in an ABCG2 protein activity assay in a membrane preparation for reducing the basal ATPase activity of said preparation.

The invention also relates to a use of a membrane preparation as defined herein in an ABCG2 protein activity assay.

The invention also relates to a use of an ABCG2 assay kit as defined herein in an ABCG2 protein activity assay.

Preferably the membrane preparation or the kit according to the invention is used for studying transporter - drug interactions.

Preferably, the assay is a high sensitivity ABCG2 ATPase assay or a substrate transport assay.

Preferably, the ABCG2 protein activity assay is a high sensitivity assay for functional characterization of the ABCG2 transporter.

Preferably, in said assay a bile acid compound as defined herein is used to reduce the basal ATPase activity.

Preferably, the modifying agents do not change the effects of substrates or inhibitors on the activity of the ABC protein.

The invention also relates to the purified and reconstituted ABCG2 containing liposomes, lipid compartments or vesicles disclosed herein or being obtainable by the purification and reconstitution method according to the invention.

According to the invention the ATPase activity of the membrane preparations is measurable ATPase activity can be stimulated or inhibited by ABCG2 substrates and inhibitors.

In a preferred embodiment substrate stimulated activity, e.g. ATPase activity or transport activity is measured. In a preferred embodiment relative substrate stimulated activity is increased by adding the bile acid compound(s) by a factor of at least 1.3, at least 1.5, at least 1.7 at least 1.9 or at least 2. In a further preferred embodiment the activity of ABCG2 measured in the membrane preparation of the invention by a substrate in the presence of the bile acid compound(s) is increased by a factor of at least 1.5, at least 2 or at least 3 or at least 4 or at least 5.

In a preferred embodiment the membrane preparations of the invention further comprise an agent for increasing transport activity, e.g. cholesterol, preferably added cholesterol or a cholesterol analogue or derivative.

Below the invention is further described by illustrative examples.

### EXAMPLES

### EXAMPLE 1 - Materials and Methods

*Generation of Recombinant Baculoviruses*-Recombinant baculovirus carrying the human wt (wild type) ABCG2 or cleavable His6 tagged ABCG2 cDNA, were generated with the BaculoGold Transfection Kit (Pharmingen) according to the manufacturer's instructions (Ozvegy-Laczka et al., 2008).

In short, pAcUW21-L/ABCG2 was constructed by removing the full-length human ABCG2 cDNA (G482 variant) from pcDNA3.1/ABCG2 with SacI digestion, and ligating the resulting fragment to the SacI site of the modified baculovirus transfer vector, pAcUW21-L (see Özvegy et al., 2001 and Ozvegy et al. 2002).

### His6-tag sequence before the N-terminal of ABCG2:

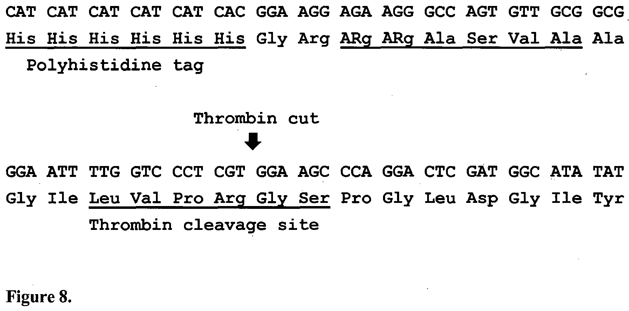

The N-terminal sequence (SEQ ID NO: 1) of the His6 tagged ABCG2 as well as its coding nucleotide sequence (SEQ ID NO: 2) is shown on Figure 8. The His6 tag and the recognition sequence of the Thrombin cleavage site are underlined and cleavage site is indicated by an arrow.

Several His6-tagged versions of the ABCG2 protein have been prepared and expressed them in Sf9 cells. It has been found that the C-terminally tagged ABCG2 was completely inactive, while the N-terminally tagged version retained full activity.

### Culturing and viral infection of Sf9 insect cells

Culturing conditions are optimized for obtaining high expression level of ABCG2. Sf9 cells are grown in spinner flasks in suspension culture, while baculovirus infection is carried out in cells placed in a roller flask for increasing protein production capacity.

Medium for insect cell culturing: Sigma TNM-FH (T1032) insect cell culture medium supplemented with 10% fetal bovine serum (Sigma F0643).

*Sf9 in spinner flask:* culturing starts at 5x10⁵ cells/ml and the cells are grown for 3 days to a density of about 15x10⁵ cells/ml.

### Amplification of baculovirus:

The amplified virus stock is produced for 7 days with 10⁷ Sf9 cell number/25 ml in a culture flask, without medium change. Polyhedra numbers are checked and virus batch is stored. The cells should contain numerous virus polyhedra, the supernatant is collected and stored at 4° C for continuous use or at -80°C as a stock.

### Sf9 infection in roller flasks (Corning 430852)

2.5-3x10⁸ cells/20 ml media is mixed with 20 ml of amplified virus stock and allowed to settle down for 3-5 hours in a roller flask, rolling at low speed (5-8 turn/min). Then the 60 ml of media is added, cells are cultured at 27°C and harvested after 72 hours (at the 4^{th} day).

Infection in suspension culture is also possible. It is advantageous for production of large quantities, but results in a relatively lower the expression level.

### Expression

It was found that the expression level in the Sf9/baculovirus system greatly exceeded that of the mammalian cell lines thus providing sufficient quantity for purification procedures. Membranes were prepared as described in (Sarkadi et al., 1992), or (Ozvegy et al., 2002) or by the method described below resulting in the production of highly active ABCG2 protein in Sf9 membrane vesicles. This preparation contains about 2-4% ABCG2 protein of the total membrane proteins, and is suitable for ABCG2-ATPase assays as well as for vesicular transport assays. It has been found that the His6-tagged ABCG2 protein shows similar expression level and ATPase or transport activity as the untagged, wild type ABCG2 protein.

### Membrane preparation

Cells are disrupted by mechanical homogenization in a glass-teflon homogenizer in a hypo-osmotic buffer (50mM TRIS, 50 mM mannitol, 2 mM EGTA pH 7.0, protease inhibitors: 8 ug/ml aprotinin, 10 ug/ml leupeptin, 0.5 ug/ml PMSF, 2 mM DTT). Homogenate is centrifuged at low speed and in a second round its supernatant is centrifuged at 50 000 x g, for 60 min. All procedure is carried out on at 4°C. The membrane pellet is homogenized in the buffer and stored at -80°C.

### Effect of cholesterol

As shown earlier by (Telbisz et al., 2007), the activity of ABCG2 is greatly stimulated by cholesterol loading of the isolated membranes. It has been found by the Inventors that the same is true for the His6-tagged ABCG2 (unpublished). Thus, for direct measurements with ABCG2 in Sf9 membrane vesicles the membrane is loaded with cholesterol. After homogenization the sample is incubated with 2.5 mg/ml cholesterol-cyclodextrin complex (Cyclolab, Budapest) for 20 minutes at +4°C then followed by centrifugation steps.

### ABCGG2 purification

Crude membranes (stored at -80°C) are pooled and washed at 4°C in high salt wash buffer (20 mM TRIS, 0.5 mM EDTA, 500 mM NaCl, 1 mM b-mercaptoethanol, pH 8.0). The membranes were centrifuged at 50000 x g for 45min.

Pellet is resuspended in a glass-teflon homogenizer in 10 ml solubilization buffer without lipid and detergent than sonicated lipid mixture is added. Solubilization buffer is composed of 20 mM TRIS pH 8.0, 500 mM NaCl, 10 % glycerol, 4 mM imidazol, 1mM b-mercaptoethanol, protease inhibitors and solubilized lipid mix of 0.4% E.coli lipid, 1% DDM (final concentrations). Membrane protein solubilization is carried out at 4°C, the sample is rotated for 1 hour and centrifuged at 100 000 x g for 45 min to obtain solubilized protein in the supernatant.

His-binding (NiNTA) affinity chromatography (Sigma His select HF Ni- affinity gel **H 0537**) is made at 4°C as batch purification allowing binding the protein overnight in a rotating bottle.

Washings are made in several steps by TRIS buffers (20 mM pH8.0, 1 mM b-mercaptoethanol, 10% glycerol, 0.01% DDM) containing first 500 mM, later 30 mM NaCl. Elution of His6-ABCG2 protein is carried by 200 mM imidazol concentration at 30 mM NaCl containing buffer (other compounds as given above) in 5 rounds. Eluted ABCG2 containing samples can be concentrated and desalted by using 100 kDa Amicon filters.

### Reconstitution of ABCG2 protein in lipid environment

Functionality highly depends on the applied lipids. In our assays the reconstitution is performed in E.coli lipid extract supplemented with cholesterol or (bovine) brain lipid extract (Avanti). Lipids are dried from chloroform solutions under N₂ and liposomes are formed in the aqueous solution by sonication.

Reconstitution can be made in higher amount where the intact liposomes (24 mg/ml) are destabilized by detergent (0.3% Triton-X), incubated with purified, concentrated ABCG2 protein for at least 30 minutes at room temperature and the excess detergent is removed in several steps (RT, 4°C) by BioBead adsorbent. The final ABCG2-liposomes (in 10mM HEPES, pH 7.5, 50mM KCl) are stored at -80°C. This reconstitution method needs a lot of proteins (several mgs) and takes longer time (days).

This type of ABCG2-liposome preparation can be applied in ATPase or transport measurements as well.

If only a smaller amount of protein is available, the method below can be applied.

A more simple way of reconstitution can be used for ATPase measurements. In this case the liposomes are made in the assay mixture in the desired concentration at the day of the measurement. Purified ABCG2 is added directly to ATPase reaction mixture containing also the liposomes, and after a brief preincubation (20 minutes) the ATPase assay can be carried out in the usual way.

### Protein quantitation

Protein quantitation in the membrane preparations was carried out by the method of Lowry. For quantitation of proteins all proteins are precipitated by 0,4% DOC (deoxycholate)-25% TCA (trichloroacetic acid). The precipitate is centrifuged and resolved in reagent mixture. Proteins are detected based on a quantitative colorimetric reaction (Folin-Ciocalteau reagent).

### PAGE and Western blotting

Samples were analysed on 7.5% SDS-Page ELFO by coomassie staining. After blotting samples ABCG2 was specifically detected by BXP-21 antibody (mouse, 1:2000) and HRP labelled anti-mouse antibody (1:20000) and developed by enchanced chemiluminescense kit (Amersham)

### ATPase activity measurement

ABCG2-Sf9 membrane vesicles (10 ug total protein) or isolated ABCG2 (0.2 ug) and separately added liposomes or reconstituted ABCG2-proteoliposome (0.2 ug) was added to assay mixture (final volume 150 ul) and preincubated for 5 or 20 minutes at 37°C when isolated ABCG2 and liposomes are used.

Reaction is started by adding Mg-ATP pH 7.0 to the reaction mixture at 3 mM and stopped after 20 or 30 minutes (for purified ABCG2) by 5% SDS.

Self-degradation of ATP is checked in each measurement by ABCG2 free samples.

*Assay mixture* was adapted for different setup with different type of ABCG2 samples. Assay buffer was 40 mM TRIS-MOPS pH 7.0 with 0.5 mM EGTA, 50 mM KCl₂ and 2 mM DTT.

For 'ABCG2 in Sf9 membrane vesicle' setup 5 mM Na-azide, 1 mM ouabain, were given to inhibit Sf9 intrinsic ATPases. ABC specific ATPase was measured as vanadate sensitive ATPase after determining the vanadate (1.3 mM) insensitive background.

For simplified version directly using the purified non-reconstituted ABCG2 samples liposomes of desired combination and concentrations of lipids were made in assay mixture.

For ABCG2-liposome samples there was no further additives.

The bile acids or bile acid derivatives are solved in distilled water or DMSO (for hidrophobic agents like cholic acid) in a 100-150x of the desired concentration and added to the ice-cold reaction mixture before starting the reaction.

### Colorimetric assay for determination of deliberated inorganic phosphate (Pi)

A concentration set of K₂HPO₄ (10-50 ug/sample) is used as standard.

150 ul of sample is mixed with 300 ul of Reagent A (2.5 M H₂SO₄, 1% ammonium molybdate, 0.014% antimony-potassium-tartarate), 700 ul of 20% acetic acid and 150 ul of 1% ascorbic acid (freshly prepared) and vortexed immediately. Absorbance of samples is measured at 800 nm at exactly after 30 minutes (see Sarkadi et al., 1992).

### EXAMPLE 2 - Preparation of purified and reconstituted, fully active ABCG2 transporter protein

Detailed biochemical and functional characterization of the membrane transporter proteins requires isolated preparations. In a preferred embodiment, the preparations are purified and reconstituted. Purification of human membrane transporters in an active form and their proper reconstitution are major problems and require a significant amount of experimentation the success of which is uncertain. As described below, we have developed a method for obtaining pure, functional ABCG2 protein from human ABCG2 expressing Sf9 insect cells. Since the appropriate lipid environment is essential for proper function of membrane proteins, the present inventors also provided a reconstitution method for obtaining purified ABCG2 protein, whereas its function is preserved in liposomes.

### Steps of the exemplary ABCG2 purification method in brief:

1. A cleavable 6 histidine tag (His6-tag) is engineered to the N-terminus of the ABCG2 protein for allowing purification. The protein is overexpressed in a baculovirus-Sf9 insect cell system. In preliminary studies the present inventors have documented the unchanged functionality of the His6-tag modified ABCG2
2. Isolated membranes are prepared from the His6-ABCG2 overexpressing Sf9 cells. The membrane preparation can be stored at -80°C and pooled.
4. The His6-ABCG2 protein is solubilized from the membrane by a detergent-lipid mixture. Solubilization was carefully optimized for allowing ABCG2 functionality.
5. The ABCG2 protein was purified by His-binding Ni-NTA affinity cromatography. The eluted fractions containing His6-ABCG2 in detergent are concentrated and can be stored at -80°C (see Fig. 1).
6. The purified ABCG2 protein is reconstituted into appropriate lipid environment by using commercially available lipid and cholesterol preparations. Reconstitution into liposomes can be successfully performed by using E.coli lipid extract supplemented with cholesterol, or a brain lipid extract. Reconstitution of a fully active ABCG2 is achieved when detergent is removed by BioBead adsorbent. The purified and reconstituted ABCG2 liposomes can be stored at -80°C. The ABCG2 proteoliposome preparation can be applied both in specific ATPase and drug transport measurements.

The purified, reconstituted ABCG2 has a relatively high drug-independent basal ATPase activity, similarly to that reported for the ABCG2 protein overexpressed in Sf9 membrane vesicles (Ozvegy et al., 2001). The basal ABCG2-ATPase activity can be stimulated or inhibited by drugs, which represent various types of drug interactions with the transporter.

We found that, similarly to the isolated Sf9 cell membrane preparations (Telbisz et al., 2007), cholesterol significantly increases the drug-stimulated ATPase activity of the isolated ABCG2 protein (Figure 2). This cholesterol effect can be observed when the ABCG2 protein is reconstituted in E. coli lipid extract, while in mammalian brain lipid extracts the presence of cholesterol provides full activity.

As documented in Fig 2, the isolated and reconstituted human ABCG2 shows a maximum ATPase activity of up to 20 moles Pi liberated / moles of ABCG2 / second, thus has a turnover rate of greatly exceeding that reported for ABCG2 in the literature (McDevitt et al., 2006; Pozza et al., 2006), and is similar to that obtained by isolated and reconstituted ABCB1 (MDR1/Pgp), recognized as fully functional preparations (Ambudkar et al., 1992; Doige et al., 1992; Shapiro and Ling, 1994; Sharom et al., 1993).

### For details and examples see the Methods section.

### EXAMPLE 3 - High sensitivity ABCG2-ATPase assay by using purified and reconstituted ABCG2, and isolated membranes containing the ABCG2 protein

A key step of the present invention is to apply modifier agents which significantly decrease the basal ABCG2-ATPase activity, without altering the drug stimulation or the drug inhibition of the transporter. This modification greatly increases the sensitivity of the assay for characterizing ABCG2 and drug interactions.

In our studies we found that such an effect can be achieved by the application of various bile acids, as an example shown in Figure 3, by cholic acid. Similar effects were obtained, although with variable efficiency and concentration dependence by taurocholate, glycocholate or taurodeoxycholate.

### max: 1,

### decrease

### to 0.2

In similar experiments we also observed that a similar effect to those by bile acids could be obtained when the detergent, CHAPS was added to the membrane preparations.

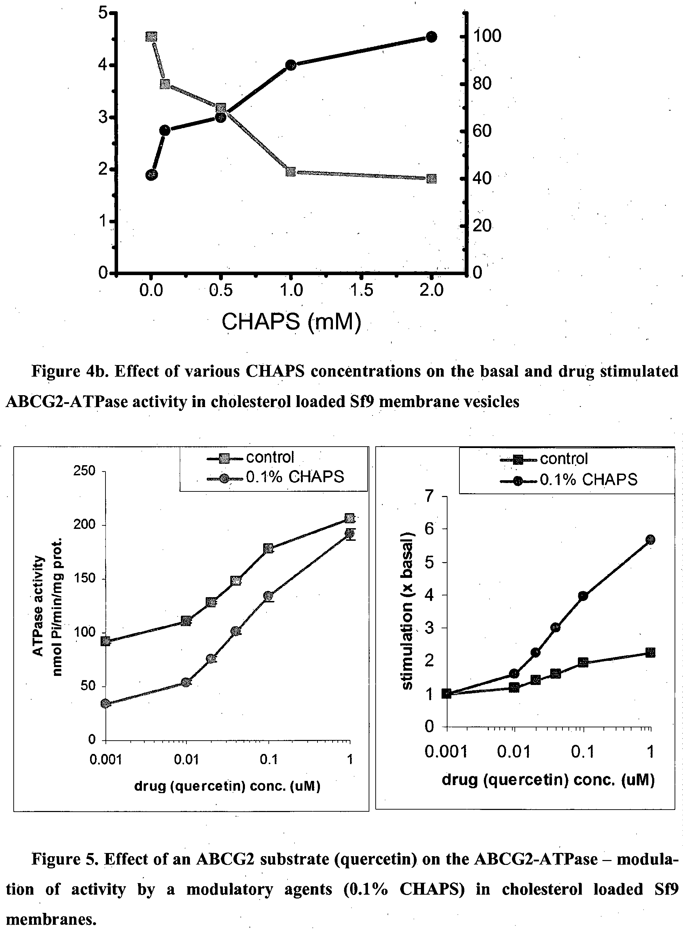

As shown in Fig. 4, CHAPS in concentrations well below the CMC, thus the detergent effects, sigificantly decreased the basal ABCG2-ATPase activity, while greatly increase the relative value of drug (e.g. quercetin) stimulation of the ATPase activity.

As a summary, the "modulatory agents", e.g. bile acids or related compounds, e.g. CHAPS, significantly decrease the basal level of the ABCG2-ATPase activity, while do not change the drug-stimulation of this ATPase activity.

In the following experiments we have studied various drug interactions with the ABCG2 multidrug transporter and the effects of modulatory agents. In most cases we present data obtained with CHAPs, a well defined and pure chemical.

As documented in Figure 5, when quercetin, an ABCG2 substrate was used to stimulate the ABCG2-ATPase in isolated Sf9 cell membrane preparations, the maximum level of stimulation was unchanged (Panel A), while the relative stimulation was greatly increased in the presence of 0.1% CHAPS, as modulatory agent.

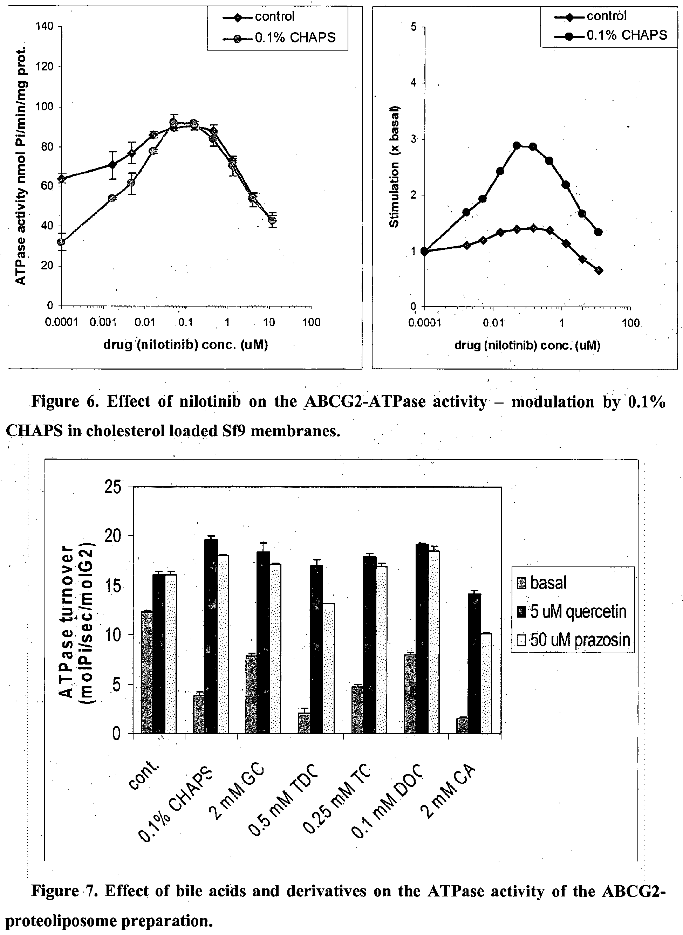

As shown in Fig. 6, the compound nilotinib has a biphasic effect on the ABCG2-ATPase activity in isolated Sf9 cell membrane preparations, showing first an activation, then an inhibition of this activity (Hegedus et al., 2009). When the nilotinib effect was measured in the presence of the modifying agent CHAPS, the maximum stimulation and the relative inhibition of the ABCG2-ATPase were unchanged. However, due to the much lower basal ATPase activity, the relative stimulation was greatly increased in the presence of 0.1% CHAPS, as modulatory agent.

**Table 1. Effect of a modulatory agent, CHAPS, on the stimulation or inhibition of the ABCG2-ATPase in cholesterol loaded, isolated Sf9 membranes by various ABCG2 interacting compounds. Stimulation is provided as the ratio of the drug-stimulated and the basal ATPase activity in the presence of maximally effective drug concentrations. Inhibition was characterized in the presence of 1 uM quercetin (providing full ATPase activity in the presence of maximally effective concentration of the inhibitors.**

| **Compound** | **Concentration in uM** | **Fold stimulation** | **Fold stimulation** |
|---|---|---|---|
| | | ***Control*** | **+ *modifying agent (0.1% CHAPS)*** |
| Quercetin | 5 | 2.0 | 5.5 |
| ZD1839 | 1 | 1.7 | 3.5 |
| Nilotinib | 0.1 | 1.6 | 3.0 |
| Topotecan | 100 | 1.1 | 1.3 |
| Flavopiridol | 50 | none | 1.8 |
| Sulfasalazine | 5 | 2.0 | 4.0 |
| Prazosin | 50 | 1.8 | 3.5 |

| | | **Inhibition** | **Inhibition** |
|---|---|---|---|
| FTC | 10 | 70% | 70% |
| Ko143 | 1 | 90% | 90% |
| Elacridar | 5 | 70% | 70% |

Table 1. shows a summary of the modulating agent CHAPS on various drug interactions with the ABCG2 multidrug transporter, measuring the ABCG2-ATPase activity. As documented, in most cases drug stimulation is greatly increased, while the drug concentrations providing maximum stimulation are unchanged. In the case of various inhibitory compounds, the inhibitory potential is unchanged in the presence of the modulatory agent. We have obtained similar data with bile acids, e.g. cholic acid (data not shown).

The ABCG2 protein has a high level basal vanadate-sensitive ATPase activity, which can be further stimulated by transported substrates, e.g. quercetin, prazosin or various TKI inhibitors, and fully inhibited by Ko143 or FTC (Ozvegy et al., 2002). Table 1 also indicates that the inhibitory effect of these inhibitors is independent of the presence of bile acid compounds, i.e. the percent inhibition is about the same in the presence and in the absence of bile acid compounds.

In the previous results we have shown data obtained with isolated membrane preparations of Sf9 cells, overexpressing the human ABCG2 protein, and loaded with cholesterol to mimick mammalian cell membranes. When using the isolated ABCG2 protein, reconstituted in cholesterol containing liposomes, we observed similar effects of the modifying agents, e.g. cholic acid or CHAPS on the ABCG2-ATPase activity.

As shown in Figure 7, the isolated and reconstituted ABCG2 protein, especially in the presence of high cholesterol (allowing full transport activity) has a relatively high basal ATPase activity, which is only slightly stimulated by the addition of substrates (e.g. quercetin or prazosin). In the presence of various modifying agents this basal activity is greatly reduced, while the maximum level of drug stimulation is practically unchanged. Therefore the sensitivity of the assay to study drug interactions is greatly increased.

### INDUSTRIAL APPLICABILITY

The present invention allows a high-sensitivity screening of drug interactions with the ABCG2 multidrug transporter and potentially with the related MDR-ABC transporters. The invention includes the purification and reconstitution of the ABCG2 protein in a fully active form and provides methods to perform the measurement of the drug-dependent ABCG2-ATPase activity with a high sensitivity.

### REFERENCES

Ambudkar, S.V., Lelong, I.H., Zhang, J., Cardarelli, C.O., Gottesman, M.M., Pastan, I., 1992. Partial purification and reconstitution of the human multidrug-resistance pump: characterization of the drug-stimulatable ATP hydrolysis. Proc Natl Acad Sci U S A 89, 8472-8476.
Dezi et al. The multidrug resistance half-transporter ABCG2 is purified as a tetramer upon selective extraction from membrane. Biochimica et Biophysica Acta (BBA) - Biomembranes, Elsevier, Amsterdam, 1798(11) 2010 2096-2101
Doige, C.A., Yu, X., Sharom, F.J., 1992. ATPase activity of partially purified P-glycoprotein from multidrug-resistant Chinese hamster ovary cells. Biochim Biophys Acta 1109, 149-160.
Hegedus, C., Ozvegy-Laczka, C., Apati, A., Magocsi, M., Nemet, K., Orfi, L., Keri, G., Katona, M., Takats, Z., Varadi, A., Szakacs, G., Sarkadi, B., 2009. Interaction of nilotinib, dasatinib and bosutinib with ABCB1 and ABCG2: implications for altered anti-cancer effects and pharmacological properties. Br J Pharmacol 158, 1153-1164.
Imai Y et al. 2003 Breast Cancer Resistance Protein Exports Sulfated Estrogens but Not Free Extrogens" Molecular Pharmacology 64, 610-618
Jacobs, A., Emmert, D., Wieschrath, S., Hrycyna, C. A. and Wiese, M. 2011 Recombinant synthesis of human ABCG2 expressed in the yeast Saccharomyces cerevisiae: an experimental methodological study. Protein J. 30, 201-211
McDevitt, C.A., Collins, R.F., Conway, M., Modok, S., Storm, J., Kerr, I.D., Ford, R.C., Callaghan, R., 2006. Purification and 3D structural analysis of oligomeric human multidrug transporter ABCG2. Structure 14, 1623-1632.
Ozvegy-Laczka, C., Laczko, R., Hegedus, C., Litman, T., Varady, G., Goda, K., Hegedus, T., Dokholyan, N.V., Sorrentino, B.P., Varadi, A., Sarkadi, B., 2008. Interaction with the 5D3 monoclonal antibody is regulated by intramolecular rearrangements but not by covalent dimer formation of the human ABCG2 multidrug transporter. J Biol Chem 283, 26059-26070.
Ozvegy, C., Litman, T., Szakacs, G., Nagy, Z., Bates, S., Varadi, A., Sarkadi, B., 2001. Functional characterization of the human multidrug transporter, ABCG2, expressed in insect cells. Biochem Biophys Res Commun 285, 111-117.
Ozvegy, C., Varadi, A., Sarkadi, B., 2002. Characterization of drug transport, ATP hydrolysis, and nucleotide trapping by the human ABCG2 multidrug transporter. Modulation of substrate specificity by a point mutation. J Biol Chem 277, 47980-47990.
Pavek P et al. 2005 Human Cancer Resistance Protein: Interactions with Steroid Drugs, Hormones, the Dietary Carcinogen 2-Amino-1-methyl-6-phenylimidazo(4,5-b)pyridine and Transport ofCimetidine 312, 144-152
Pozza, A., Perez-Victoria, J.M., Sardo, A., Ahmed-Belkacem, A., Di Pietro, A., 2006. Purification of breast cancer resistance protein ABCG2 and role of arginine-482. Cell Mol Life Sci 63, 1912-1922.
Sarkadi, B., Price, E.M., Boucher, R.C., Germann, U.A., Scarborough, G.A., 1992. Expression of the human multidrug resistance cDNA in insect cells generates a high activity drug-stimulated membrane ATPase. J Biol Chem 267, 4854-4858.
Shapiro, A.B., Ling, V., 1994. ATPase activity of purified and reconstituted P-glycoprotein from Chinese hamster ovary cells. J Biol Chem 269, 3745-3754.
Sharom, F.J., Yu, X., Doige, C.A., 1993. Functional reconstitution of drug transport and ATPase activity in proteoliposomes containing partially purified P-glycoprotein. J Biol Chem 268, 24197-24202.
Telbisz, A., Muller, M., Ozvegy-Laczka, C., Homolya, L., Szente, L., Varadi, A., Sarkadi, B., 2007. Membrane cholesterol selectively modulates the activity of the human ABCG2 multidrug transporter. Biochim Biophys Acta 1768,2698-2713.
Vaidya S et al. Lack of interaction between tauroursodeoxycholate and ATP-binding cassette transporter isoform G2 (ABCG2). Molecular Pharmaceutics, 3(3) 2006 303-306]
Velamakanni S. et al. 2008. A functional steroid-binding element in an ATP-binding cassette multidrug transporter" Molecular Pharmacology 73 12-17

## Claims

1. Use of a kit in an increased sensitivity ABCG2 protein activity assay, said kit comprising
- a membrane preparation or liposomes, said membranes or liposomes comprising a recombinant ABCG2 transporter protein capable of transporting an ABCG2 substrate compound across the membrane and showing substrate-stimulated ATPase activity,
- one or more bile acid compounds,
wherein said membrane preparation is a cell membrane preparation or a membrane protein preparation reconstituted in liposomes, wherein the basal ATPase activity of the ABCG2 protein is reduced in said assay in comparison with a control not comprising said one or more bile acid compounds.

2. The use of a kit of any of the previous claims wherein said membrane preparation is a cell membrane preparation obtainable from cells expressing said recombinant ABCG2 preferably by a method according to any of claims 12 to 15,
said preparation comprising a dimeric ABCG2 having a substrate transport activity.

3. The use of a kit of any of claims 1 to 2 wherein said membrane preparation is a membrane protein preparation comprising a reconstituted ABCG2 protein.

4. The use of a kit of claim 3 wherein said membrane protein preparation is obtainable by a method comprising the following steps:
- providing or preparing an expression vector suitable for expressing a nucleic acid in a host, said nucleic acid comprising a nucleotide sequence encoding an ABCG2 protein and, operably linked thereto a nucleotide sequence encoding a peptide tag for protein purification.
- overexpressing said nucleic acid sequence in a host to obtain a tagged protein comprising the ABCG2 protein and the peptide tag, wherein said ABCG2 protein is functional,
- obtaining the expressed tagged protein in a solubilized form,
- the solubilized tagged protein is subjected to purification comprising affinity chromatography step specific for the peptide tag to obtain a purified protein,
- the purified protein is reconstituted into an appropriate lipid environment for regaining function, preferably into liposomes,
wherein
the affinity chromatograpy is metal chelate chromatograpy and
the peptide tag is capable of complexing the metal ion of the metal chelate chromatography and is engineered to the N-terminus of the ABCG2 protein for allowing purification.

5. Use of a membrane preparation in an increased sensitivity ABCG2 protein activity assay, said preparation comprising
- liposomes, said liposomes comprising a recombinant ABCG2 transporter protein capable of transporting an ABCG2 substrate compound across the membrane of said liposomes and showing substrate-stimulated ATPase activity,
- one or more bile acid compounds,
wherein the level of said bile acid derivative in said preparation is below the critical micelle forming concentration (CMC) and
wherein said membrane preparation is a cell membrane preparation or a membrane protein preparation, and
wherein the basal ATPase activity of the ABCG2 protein is reduced in said assay in comparison with a control not comprising said one or more bile acid compounds.

6. Use of a membrane preparation of claim 5 wherein the basal ATPase activity of the membrane preparation is lower than the basal ATPase activity of a control preparation lacking said one or more added bile acid compounds or comprising a lower level thereof.

7. An ABCG2 protein activity assay method comprising the steps of
- providing a membrane preparation as defined in any of claims 5 to 6, said membrane preparation comprising one or more bile acid compounds
- assessing the activity of the ABCG2 protein present in the membrane preparation, wherein
the basal ATPase activity of the ABCG2 protein is reduced in comparison with a control not comprising said one or more bile acid compounds.

8. Use of a bile acid compound as defined in any of claims 1 to 5 for reducing basal ATPase activity of a membrane preparation comprising a recombinant ABCG2 transporter protein, capable of transporting an ABCG2 substrate compound across the membrane,
wherein said membrane preparation is a cell membrane preparation or a membrane protein preparation reconstituted in liposomes.

9. Use of a membrane preparation as defined in any of claims 5 to 6 in an ABCG2 protein activity assay wherein the basal ATPase activity of the ABCG2 protein is reduced in said assay in comparison with a control not comprising said one or more bile acid compounds wherein
the one or more bile acid compound are selected from cholic acid, chenodeoxycholic acid, glycocholic acid, taurocholic acid, deoxycholic acid, lithocholic acid or a derivative thereof which is an amide or an ester of the carboxyl group on the side chain connected to the 17 position of the sterane core or a salt thereof.

10. The use according to any of claims 1 to 9, said kit as defined in any of claims 1 to 4 or said membrane preparation as defined in any of claims 5 to 6 further comprising an agent for increasing transport activity, preferably cholesterol.

11. A method for obtaining an isolated membrane protein preparation for use in an ABCG2 assay according to claim 7, said method comprising
- providing or preparing an expression vector suitable for expressing a nucleic acid in a host, said nucleic acid comprising a nucleotide sequence encoding an ABCG2 protein and, operably linked thereto a nucleotide sequence encoding a peptide tag for protein purification.
- overexpressing said nucleic acid sequence in a host to obtain a tagged protein comprising the ABCG2 protein and the peptide tag, wherein said ABCG2 protein is functional,
- obtaining the expressed tagged protein in a solubilized form by
- preparing cell membrane fraction from the cells of the host, said membranes comprising the tagged protein,
- solubilizing the tagged protein from the cell membrane fraction by a detergent-lipid mixture whereas the functionality of the ABCG2 transporter is maintained, in a solubilisation buffer by DDM detergent and E.coli lipid mixture and
- the solution fraction is separated to obtain solubilised ABCG2 protein in the supernatant,
- the solubilized tagged protein is subjected to purification comprising affinity chromatography step specific for the peptide tag to obtain a purified protein,
- the purified protein is reconstituted into an appropriate lipid environment for regaining function with preformed liposomes made of a lipid extract, said lipid extract being selected from the group of
a bacterial lipid extract and/or
a eukaryotic lipid extract selected from a vertebrate lipid extract, a mammalian lipid extract and a brain lipid extract,
wherein preferably the reconstitution lipid extract is supplemented with cholesterol, and
- wherein the dimeric form of ABCG2 is maintained and this fact is detected by a method assessing the size of the native proteins, and
wherein
said host is an insect cell,
the affinity chromatograpy is metal chelate chromatograpy and
the peptide tag is capable of complexing the metal ion of the metal chelate chromatography and is engineered to the N-terminus of the ABCG2 protein for allowing purification.

12. The method according to claim 11 wherein the peptide tag is a His-tag.

13. The method according to claim 11 wherein the preformed liposomes in the reconstitution agent are destabilized and the reconstitution agent is incubated with purified ABCG2 protein, wherein preferably the reconstitution lipid extract is supplemented with cholesterol.

14. The method according to claim 11 wherein the step of obtaining the expressed tagged protein in a solubilized form comprises
- preparing cell membrane fraction from the cells of the host by
- disruption of the cells by mechanical homogenization in a hypo-osmotic buffer,
- removal of cellular debris by low speed centrifugation at 200 to 400 g
- centrifugation of the supernatant to obtain a membrane pellet at 30 000 to 70 000 g for 30 min to 90 min,
- homogenization of the membrane pellet,
said membranes comprising the tagged protein,
- solubilizing the tagged protein from the cell membrane fraction by a detergent-lipid mixture whereas the functionality of the ABCG2 transporter is maintained,
- assessing the amount of protein and/or transporter activity in the membrane preparation, and optionally
- washing the pelleted membranes,
- repeatedly pelleting, washing and resuspending the membranes.

## Patentansprüche

1. Verwendung eines Kits in einem ABCG2-Proteinaktivitätstest mit erhöhter Empfindlichkeit, wobei das erwähnte Kit enthält
- ein Membranpräparat oder Liposomen, wobei die erwähnten Membranen und Liposomen ein rekombinantes ABCG2-Transporterprotein enthalten, welches geeignet ist, die ABCG2-Substratverbindung über die Membran zu transportieren und substratstimulierte ATPase-Aktivität zu zeigen,
- eine oder mehrere Gallensäureverbindungen,
wobei das erwähnte Membranpräparat ein Zellmembranpräparat oder ein Membranproteinpräparat ist, welches in Liposomen rekonstituiert ist, wobei die basale ATPase-Aktivität des ABCG2-Proteins in dem erwähnten Test im Vergleich mit einer Kontrolle ohne die erwähnten eine oder mehreren Gallensäureverbindungen reduziert ist.

2. Die Verwendung eines Kits nach einem der vorherigen Ansprüche, wobei das erwähnte Membranpräparat ein Zellmembranpräparat ist, welches aus das erwähnte rekombinante ABCG2 expressierenden Zellen, bevorzugt durch ein Verfahren nach einem der Ansprüche 12 bis 15, herstellbar ist,
wobei das erwähnte Präparat ein dimeres ABCG2 mit Substrattransportaktivität enthält.

3. Die Verwendung eines Kits nach einem der Ansprüche 1 bis 2, wobei das erwähnte Membranpräparat ein rekonstituiertes ABCG2-Protein enthaltendes Membranproteinpräparat ist.

4. Die Verwendung eines Kits nach Anspruch 3, wobei das erwähnte Membranproteinpräparat durch ein Verfahren herstellbar ist, enthaltend die folgenden Schritte:
- Bereitstellung oder Herstellung eines Expressionsvektors, welcher zum Expressieren einer Nukleinsäure in einem Host geeignet ist, welche Nukleinsäure eine ein ABCG2-Protein entkodierende Nukleotidsequenz und eine mit diesem operativ verbundene, eine Peptid-Markierung zur Proteinreinigung entkodierende Nukleotidsequenz enthält,
- Überexpressierung der erwähnten Nukleotidsequenz im Host zur Gewinnung eines markierten Proteins, welches das ABCG2-Protein und die Peptid-Markierung enthält, wobei das erwähnte ABCG2-Protein funktionell ist,
- Erhalten des markierten Proteins in solubilisierter Form,
- das solubilisierte markierte Protein ist einer Reinigung unterworfen, die einen Affinitätschromatographie-Schritt beinhaltet, welcher für die Peptid-Markierung spezifisch ist, um gereinigtes Protein zu gewinnen,
- das gereinigte Protein ist in eine Lipidumgebung zur Wiederherstellung der Funktionalität, bevorzugt in Liposomen, rekonstituiert,
wobei
- die Affinitätschromatographie eine Metallchelatchromatographie ist, und
- die Peptid-Markierung zur Komplexierung des Metallions von Metallchelatchromatographie geeignet ist, und zum N-Terminus des ABCG2-Proteins entwickelt ist, um die Reinigung zu ermöglichen.

5. Verwendung eines Membranpräparats in einem ABCG2-Proteinaktivitätstest mit erhöhter Empfindlichkeit, wobei das erwähnte Membranpräparat enthält
- Liposomen, wobei die erwähnten Liposomen ein rekombinantes ABCG2-Transporterprotein enthalten, welches geeignet ist, um eine ABCG2-Substratverbindung über die Membran der erwähnten Liposomen zu transportieren und substratstimulierte ATPase-Aktivität zu zeigen,
- eine oder mehrere Gallensäureverbindungen,
wobei das Niveau der erwähnten Gallensäure-Derivate im erwähnten Präparat unter der kritischen Mizellbildungskonzentration (CMC) liegt, und
wobei das erwähnte Membranpräparat ein Zellmembranpräparat oder ein Membranproteinpräparat ist, und wobei die basale ATPase-Aktivität des ABCG2-Proteins in dem erwähnten Test im Vergleich mit einer Kontrolle ohne die erwähnten eine oder mehreren Gallensäureverbindungen reduziert ist.

6. Verwendung eines Membranpräparates nach Anspruch 5, wobei die basale ATPase-Aktivität des Membranpräparates kleiner ist als die basale ATPase-Aktivität eines Kontrollpräparates ohne die erwähnten eine oder mehreren zugegebenen Gallensäureverbindungen oder mit einem erniedrigten Niveau dieser.

7. Eine ABCG2-Proteinaktivitätstestmethode enthaltend die folgenden Schritte:
- Bereitstellung eines Membranpräparates definiert in einem der Ansprüche 5 bis 6, welches Membranpräparat eine oder mehrere Gallensäureverbindungen enthält,
- Feststellen der Aktivität des im Membranpräparat vorhandenen ABCG2-Proteins, wobei die basale ATPase-Aktivität des ABCG2-Proteins im Vergleich mit einer Kontrolle ohne die erwähnten eine oder mehreren Gallensäureverbindungen reduziert ist.

8. Verwendung einer Gallensäureverbindung definiert in einem der Ansprüche 1 bis 5 zur Reduzierung der basalen ATPase-Aktivität eines Membranpräparates enthaltend ein rekombinantes ABCG2-Transporterprotein, welches zum Transport einer ABCG2-Substratverbindung über die Membran geeignet ist,
wobei das erwähnte Membranpräparat ein in Liposomen rekonstituiertes Zellmembranpräparat oder ein Membranproteinpräparat ist.

9. Verwendung eines Membranpräparates definiert in einem der Ansprüche 5 bis 6 in einem ABCG2-Proteinaktivitätstest, wobei die basale ATPase-Aktivität des ABCG2-Proteins in dem erwähnten Test im Vergleich mit einer Kontrolle ohne die erwähnten eine oder mehreren Gallensäureverbindungen reduziert ist, wobei
die eine oder mehrere Gallensäureverbindungen aus Cholinsäure, Chenodeoxycholinsäure, Glykocholinsäure, Taurocholinsäure, Deoxycholinsäure, Lithocholinsäure oder einem Derivat dieser, welches ein Amid oder Ester der Karbonsäuregruppe an der in Position 17 des Sterankernes gebundenen Seitenkette oder dessen Salz ist, gewählt ist.

10. Die Verwendung nach einem der Ansprüche 1 bis 9, wobei das erwähnte Kit definiert in einem der Ansprüche 1 bis 4 oder das erwähnte Membranpräparat definiert in einem der Ansprüche 5 bis 6 weiterhin ein Mittel zur Erhöhung der Transportaktivität, bevorzugt Cholesterin, enthält.

11. Verfahren zur Herstellung eines isolierten Membranproteinpräparates zur Verwendung in einem ABCG2-Test nach Anspruch 7, welches Verfahren enthält
- Bereitstellung oder Herstellung eines Expressionsvektors, welcher zum Expressieren einer Nukleinsäure in einem Host geeignet ist, welche Nukleinsäure eine ein ABCG2-Protein entkodierende Nukleotidsequenz und eine mit diesem operativ verbundene, eine Peptid-Markierung zur Proteinreinigung entkodierende Nukleotidsequenz enthält,
- Überexpressierung der erwähnten Nukleotidsequenz im Host zur Gewinnung eines markierten Proteins, welches ABCG2-Protein und eine Peptid-Markierung enthält, wobei das erwähnte ABCG2-Protein funktionell ist,
- Erhalten des markierten Proteins in solubilisierter Form durch
- Herstellung einer Zellmembranfraktion von Zellen des Hosts, wobei die erwähnten Membranen das markierte Protein enthalten,
- Solubilisierung des markierten Proteins von der Zellmembranfraktion mit einem Gemisch von Detergent und Lipid,
wodurch die Funktionalität des ABCG2-Transporters in einem Solubilisierungspuffer durch ein Gemisch von DDM Detergent und E. coli Lipid aufrechterhalten ist, und
- die Solubilisierungsfraktion ist separiert zur Gewinnung eines in Überstand solubilisierten ABCG2-Proteins,
- das solubilisierte markierte Protein ist einer Reinigung unterworfen, die einen Affinitätschromatographie-Schritt beinhaltet, welcher für die Peptid-Markierung spezifisch ist, um gereinigtes Protein zu gewinnen,
- das gereinigte Protein ist in eine entsprechende Lipidumgebung zur Wiederherstellung der Funktionalität mit vorgeformten Liposomen rekonstituiert, die aus einem Lipidextrakt gewonnen sind, wobei der erwähnte Lipidextrakt aus einer Gruppe enthaltend
einen bakteriellen Lipidextrakt und/oder
einen eukaryotischen Lipidextrakt, ausgewählt aus vertebratem Lipidextrakt, mammalem Lipidextrakt und Gehirnlipidextrakt, gewählt ist,
wobei der rekonstituierende Lipidextrakt bevorzugt durch Cholesterin ergänzt ist, und
- wobei die dimere Form von ABCG2 aufrechterhalten ist, und diese Tatsache durch ein die Größe des nativen Proteins feststellendes Verfahren gemessen wird, und
wobei
der erwähnte Host eine Insektenzelle ist,
die Affinitätschromatographie eine Metallchelatchromatographie ist, und
die Peptid-Markierung zur Komplexierung des Metallions von Metallchelatchromatographie geeignet ist, und zum N-Terminus des ABCG2 Proteins entwickelt ist, um die Reinigung zu ermöglichen.

12. Das Verfahren nach Anspruch 11, wobei die Peptidmarkierung eine His-Markierung ist.

13. Das Verfahren nach Anspruch 11, wobei die vorgeformten Liposomen in dem rekonstituierenden Mittel destabilisiert sind, und das rekonstituierende Mittel mit gereinigtem ABCG2-Protein inkubiert ist, wobei der rekonstituierende Lipidextrakt bevorzugt durch Cholesterin ergänzt ist.

14. Das Verfahren nach Anspruch 11, wobei der Schritt für das Erhalten des markierten Proteins in solubilisierter Form enthält
- Herstellung einer Zellmembranfraktion von Zellen des Hosts durch
- Zerstörung der Zellen durch mechanische Homogenisierung in hypoosmotischem Puffer,
- Entfernung der Zellsplitter durch Niedergeschwindigkeitszentrifugation bei 200 bis 400 g,
- Zentrifugieren des Überstandes zur Gewinnung eines Membranpellets bei 30 000 bis 70 000 g über eine Zeitdauer von 30 Minuten bis 90 Minuten,
- Homogenisierung des Membranpellets,
wobei das erwähnte Membranpellet das markierte Protein enthält,
- Solubilisierung des markierten Proteins von der Zellmembranfraktion mit einem Gemisch von Detergent und Lipid, wodurch die Funktionalität des ABCG2-Transporters aufrechterhalten ist,
- Feststellung der Mengen des Proteins und/oder der Transporteraktivität in dem Membranpräparat, und gegebenenfalls
- Waschen der pelettierten Membranen,
- erneute Pelletierung, Waschen und Resuspendierung der Membranen.

## Revendications

1. Utilisation d'un kit dans un essai de l'activité de la protéine ABCG2 à sensibilité accrue, ledit kit comprenant
- une préparation de membrane ou des liposomes, lesdites membranes ou liposomes comprenant une protéine transporteuse ABCG2 recombinante capable de transporter un composé substrat de l'ABCG2 à travers la membrane et montrant une activité ATPase stimulée par le substrat,
- un ou plusieurs composés d'acide biliaire,
dans laquelle ladite préparation de membrane est une préparation de membrane cellulaire ou une préparation de protéine membranaire reconstituée dans des liposomes, dans laquelle l'activité ATPase basale de la protéine ABCG2 est réduite dans ledit essai en comparaison avec un témoin ne comprenant pas ledit ou lesdits composés d'acide biliaire.

2. L'utilisation d'un kit selon l'une quelconque des revendications précédentes, dans laquelle ladite préparation de membrane est une préparation de membrane cellulaire pouvant être obtenue à partir de cellules exprimant ledit ABCG2 recombinant de préférence par un procédé selon l'une quelconque des revendications 12 à 15,
ladite préparation comprenant un ABCG2 dimérique ayant une activité de transport de substrat.

3. L'utilisation d'un kit selon l'une quelconque des revendications 1 à 2, dans laquelle ladite préparation de membrane est une préparation de protéine membranaire comprenant une protéine ABCG2 reconstituée.

4. L'utilisation d'un kit selon la revendication 3, dans laquelle ladite préparation de protéine membranaire peut être obtenue par un procédé comprenant les étapes suivantes :
- fournir ou préparer un vecteur d'expression approprié pour exprimer un acide nucléique dans un hôte, ledit acide nucléique comprenant une séquence nucléotidique codant pour une protéine ABCG2 et, liée de manière opérationnelle à celle-ci, une séquence nucléotidique codant pour une étiquette peptidique pour la purification des protéines,
- surexprimer ladite séquence d'acide nucléique dans un hôte pour obtenir une protéine étiquetée comprenant la protéine ABCG2 et l'étiquette peptidique, ladite protéine ABCG2 étant fonctionnelle,
- obtenir la protéine étiquetée exprimée sous une forme solubilisée,
- la protéine étiquetée solubilisée est soumise à une purification comprenant une étape de chromatographie d'affinité spécifique pour l'étiquette peptidique pour obtenir une protéine purifiée,
- la protéine purifiée est reconstituée dans un environnement lipidique approprié pour récupérer la fonction, de préférence dans des liposomes,
dans laquelle
la chromatographie d'affinité est la chromatographie de chélate de métal, et
l'étiquette peptidique est capable de complexer l'ion métallique de la chromatographie de chélate de métal et est manipulée à l'extrémité N-terminale de la protéine ABCG2 pour permettre la purification.

5. L'utilisation d'une préparation de membrane dans un essai de l'activité de la protéine ABCG2 à sensibilité accrue, ladite préparation comprenant
- des liposomes, lesdits liposomes comprenant une protéine transporteuse ABCG2 recombinante capable de transporter un composé substrat de l'ABCG2 à travers la membrane desdits liposomes et montrant une activité d'ATPase stimulée par le substrat,
- un ou plusieurs composés d'acide biliaire,
dans laquelle le niveau dudit dérivé d'acide biliaire dans ladite préparation est inférieur à la concentration micellaire critique (CMC) et
dans laquelle ladite préparation de membrane est une préparation de membrane cellulaire ou une préparation de protéine membranaire, et
dans laquelle l'activité ATPase basale de la protéine ABCG2 est réduite dans ledit essai en comparaison avec un témoin ne comprenant pas ledit ou lesdits composés d'acide biliaire.

6. L'utilisation d'une préparation de membrane selon la revendication 5, dans laquelle l'activité ATPase basale de la préparation de membrane est inférieure à l'activité ATPase basale d'une préparation témoin dépourvue dudit ou desdits composés d'acide biliaire ajoutés ou comprenant un niveau inférieur de ceux-ci.

7. Méthode d'essai de l'activité de la protéine ABCG2 comprenant les étapes consistant à:
- fournir une préparation de membrane telle que définie dans l'une quelconque des revendications 5 à 6, ladite préparation de membrane comprenant un ou plusieurs composés d'acide biliaire
- évaluer l'activité de la protéine ABCG2 présente dans la préparation de membrane, dans lequel
l'activité ATPase basale de la protéine ABCG2 est réduite en comparaison avec un témoin ne comprenant pas ledit ou lesdits composés d'acide biliaire.

8. Utilisation d'un composé d'acide biliaire tel que défini dans l'une quelconque des revendications 1 à 5 pour réduire l'activité ATPase basale d'une préparation de membrane comprenant une protéine transporteuse ABCG2 recombinante, capable de transporter un composé substrat de l'ABCG2 à travers la membrane,
dans laquelle ladite préparation de membrane est une préparation de membrane cellulaire ou une préparation de protéine membranaire reconstituée dans des liposomes.

9. Utilisation d'une préparation de membrane selon l'une quelconque des revendications 5 à 6 dans un essai de l'activité de la protéine ABCG2 dans laquelle l'activité d'ATPase basale de la protéine ABCG2 est réduite dans ledit essai en comparaison avec un témoin ne comprenant pas ledit ou lesdits composés d'acide biliaire dans laquelle
l'un ou plusieurs composés d'acide biliaire sont choisis parmi l'acide cholique, l'acide chénodésoxycholique, l'acide glycocholique, l'acide taurocholique, l'acide désoxycholique, l'acide lithocholique ou un dérivé de ceux-ci qui est un amide ou un ester du groupe carboxyle sur la chaîne latérale connectée à la position 17 du noyau de stérane ou un sel de celui-ci.

10. L'utilisation selon l'une quelconque des revendications 1 à 9, ledit kit tel que défini dans l'une quelconque des revendications 1 à 4 ou ladite préparation de membrane telle que définie dans l'une quelconque des revendications 5 à 6 comprenant en outre un agent pour augmenter l'activité de transport, de préférence le cholestérol.

11. Procédé pour obtenir une préparation de protéine membranaire isolée pour son utilisation dans un essai d'ABCG2 selon la revendication 7, ledit procédé comprenant
- fournir ou préparer un vecteur d'expression approprié pour exprimer un acide nucléique dans un hôte, ledit acide nucléique comprenant une séquence nucléotidique codant pour une protéine ABCG2 et, liée de manière opérationnelle à celle-ci, une séquence nucléotidique codant pour un étiquette peptidique pour la purification des protéines,
- surexprimer ladite séquence d'acide nucléique dans un hôte pour obtenir une protéine étiquetée comprenant la protéine ABCG2 et l'étiquette peptidique, ladite protéine ABCG2 étant fonctionnelle,
- obtenir la protéine étiquetée exprimée sous une forme solubilisée, par
- préparer une fraction de membrane cellulaire à partir des cellules de l'hôte, lesdites membranes comprenant la protéine étiquetée,
- solubiliser la protéine étiquetée à partir de la fraction de membrane cellulaire par un mélange détergent-lipide tandis que la fonctionnalité du transporteur ABCG2 est maintenue, dans un tampon de solubilisation par un mélange de détergent DDM et de lipides de E. coli et
- la fraction en solution est séparée pour obtenir la protéine ABCG2 solubilisée dans le surnageant,
- la protéine étiquetée solubilisée est soumise à une purification comprenant une étape de chromatographie d'affinité spécifique pour l'étiquette peptidique pour obtenir une protéine purifiée,
- la protéine purifiée est reconstituée dans un environnement lipidique approprié pour récupérer la fonction, avec des liposomes préformés constitués d'un extrait lipidique, ledit extrait lipidique étant choisi dans le groupe comprenant
un extrait lipidique bactérien et / ou
un extrait lipidique eucaryotique choisi parmi un extrait lipidique de vertébré, un extrait lipidique de mammifère et un extrait lipidique cérébral,
dans lequel de préférence l'extrait lipidique de reconstitution est additionné de cholestérol, et
- dans lequel la forme dimérique d'ABCG2 est maintenue et ce fait est détecté par une méthode d'évaluation de la taille des protéines natives, et
dans lequel
ledit hôte est une cellule d'insecte,
la chromatographie d'affinité est la chromatographie de chélate de métal, et
l'étiquette peptidique est capable de complexer l'ion métallique de la chromatographie de chélate de métal et est manipulée à l'extrémité N-terminale de la protéine ABCG2 pour permettre la purification.

12. Le procédé selon la revendication 11, dans lequel l'étiquette peptidique est une étiquette His.

13. Le procédé selon la revendication 11, dans lequel les liposomes préformés dans l'agent de reconstitution sont déstabilisés, et l'agent de reconstitution est incubé avec une protéine ABCG2 purifiée, dans laquelle de préférence l'extrait lipidique de reconstitution est additionné de cholestérol.

14. Le procédé selon la revendication 11, dans lequel l'étape pour obtenir la protéine étiquetée exprimée sous une forme solubilisée comprend
- préparer une fraction de membrane cellulaire à partir des cellules de l'hôte par
- rupture des cellules par homogénéisation mécanique dans un tampon hypo-osmotique,
- élimination des débris cellulaires par centrifugation à basse vitesse entre 200 et 400 g,
- centrifugation du surnageant pour obtenir un culot membranaire à 30 000 à 70 000 g pendant 30 min à 90 min,
- homogénéisation du culot membranaire,
lesdites membranes comprenant la protéine étiquetée,
- solubiliser la protéine étiquetée à partir de la fraction de membrane cellulaire par un mélange détergent-lipide tandis que la fonctionnalité du transporteur ABCG2 est maintenue,
- évaluer la quantité de la protéine et / ou l'activité transporteuse dans la préparation de membrane, et éventuellement
- laver les membranes culottées,
- culotter de façon répétée, laver et remettre en suspension des membranes.
